**Europäisches Patentamt**

**European Patent Office**

**Office européen des brevets**

(11) Publication number : **0 519 866 A1**

# EUROPEAN PATENT APPLICATION

(21) Application number : **92810445.4**

(22) Date of filing : **10.06.92**

(51) Int. Cl.$^5$ : **C12N 15/13, C12P 21/08, C12N 15/63, C12N 5/10, C12N 5/20, A61K 39/42, G01N 33/569, G01N 33/577**

The applicant has filed a statement in accordance with Rule 28 (4) EPC (issue of a sample only to an expert). Accession number(s) of the deposit(s): ECACC 91040320, 91040321, 91052905 and 91052906.

(30) Priority : **18.06.91 EP 91810468**

(43) Date of publication of application :
**23.12.92 Bulletin 92/52**

(84) Designated Contracting States :
**AT BE CH DE DK ES FR GB GR IT LI LU NL PT SE**

(71) Applicant : **CIBA-GEIGY AG**
**Klybeckstrasse 141**
**CH-4002 Basel (CH)**

(72) Inventor : **Mestan, Jürgen, Dr.**
**Drosselweg 8**
**W-7819 Denzlingen (DE)**
Inventor : **Lazdins, Janis K., Dr.**
**Martinsgasse 13**
**CH-4051 Basle (CH)**
Inventor : **Woods-Cook, Kathie A.**
**Maulbeerstrasse 65**
**CH-4058 Basle (CH)**
Inventor : **Hardman, Norman, Dr.**
**Gstaltenrainweg 67/3**
**CH-4125 Riehen (CH)**
Inventor : **Hochkeppel, Heinz-Kurt, Dr.**
**Traugott Meyer-Strasse 1**
**CH-4147 Aesch (CH)**

(54) **Novel anti-HIV antibodies.**

(57)     The invention concerns monoclonal antibodies and antibody derivatives directed against HIV core protein p24 which recognize p24 expressed on the surface of HIV-infected macrophages and/or kill HIV-infected cells. The monoclonal antibodies of the invention may be murine antibodies or chimeric antibodies consisting of human constant regions and murine variable or hypervariable regions. Methods of manufacture of such antibodies, hybridoma or transfectoma cell lines secreting them and methods for production of the hybridoma or transfectoma cell lines are also encompassed by this invention. The invention further concerns recombinant DNA comprising an insert coding for the variable regions of antibodies against HIV core protein p24 having the mentioned properties, methods of manufacture of such recombinant DNAs, and host cells transformed with such recombinant DNAs.

    The antibodies are especially useful for the prevention of the progression of AIDS and for the treatment of AMS, but can also be used for the diagnosis of HIV infection in an immunoassay.

EP 0 519 866 A1

## Background of the invention

HIV (human immunodeficiency virus, also called lymphadenopathy virus, LAV) is the third known retrovirus of the family of human T lymphotropic retroviruses (HTLV-III) and is the causative agent of the acquired immuno defiency syndrome (AIDS) and AIDS-related conditions (ARC) preceeding the outbreak of the disease. The primary targets of HIV in the human body are specific subpopulations of T-cells, the T4 lymphocytes. Depletion of T4 lymphocytes results in a severe immunodeficiency associated with secondary infections and cancers.

The entire genome of the HIV has been sequenced, and the functions of the various genes elucidated. The major core proteins are encoded for by the *gag* gene. A *gag* precursor protein p55 is processed by a protease encoded for by the *pol* gene, and the major core protein p24 (sometimes also named p25) and proteins p18 (p17) and p13 (p 15) are produced. The *env* gene codes for the glycosylated protein gp160 which is processed to the external gp120 and the transmembrane glycoprotein gp41.

Humans infected with human immunodeficiency virus (HIV) rapidly develop high titres of antibodies against viral antigens gp120, gp160, gp41 and p24 (p25). Serum antibodies against p24 appear early in the infection, but within six months to one year before the transition from the asymptomatic to the symptomatic form of the disease (AIDS) a decrease in anti-p24 antibodies is frequently observed. This decrease is not paralleled with significant changes in anti-gp120/160 plasma antibody levels, which are maintained throughout the course of the infection until the terminal stage of the disease when the immune system is severely depressed.

Murine and human monoclonal antibodies to p24 (p25) are known. U.S. Patent 4 843 011 discloses murine monoclonal antibodies to the HIV transmembrane envelope glycoprotein gp41, the major core protein p24 and the p17 protein, and their use in immunoassays for the determination of HIV. European Patent Application EP-A 0 248 534 likewise describes a method of detection of AMS virus infection using murine monoclonal antibodies reacting with the major core protein p24. Other murine monoclonal anti-p24 (p25) antibodies are disclosed in European Patent Applications EP-A 0 211 022, EP-A 0 290 893, EP-A 0 345 461, in F. di Marzo Veronese et al., Proc. Natl. Acad. Sci. USA 82, 5199 (1985), and others. Human monoclonal anti-p24 (p25) antibodies are described by M.K. Goory et al., Proc. Natl. Acad. Sci. USA 86, 1624 (1989), and in PCT Application WO 90/09805.

Effective treatment of HIV infection is not yet possible, although a great deal of effort and resources have been directed to the development and testing of agents with anti-retroviral activity. Use of monoclonal anti-HIV antibodies has been considered for the prophylaxis and treatment of AIDS. However, application of the known anti-p24 (p25) has not turned out to be feasible since these antibodies have not been able to prevent the production of HIV particles by HIV-infected cells and to mediate selective destruction of HIV infected cells.

One major limitation in the use of murine monoclonal antibodies as in vivo diagnostic and therapeutic agents is their immunogenicity as foreign proteins, their rather long persistence in the circulation, and the formation of damaging immune complexes. On the other hand, the treatment with human monoclonal antibodies is also limited since human hybridoma cell lines are generally unstable and do not produce monoclonal antibodies of appropriate specificity in sufficient quantities and at reasonable costs. A promising alternative is the modification of immunoglobulin genes in order to tailor monoclonal antibodies for particular diagnostic and therapeutic tasks. Due to the fact that the variable region and each of the constant region domains of immunoglobulin molecules are encoded in separate exons with their own splice sites, recombinant DNA techniques can be used to isolate different parts of cloned immunoglobulin genes and ligate them to parts of other immunoglobulins. The reconstructed genes are expressed by appropriate transformed continuous cell lines. Murine antibodies can, for example, be converted into "humanized" antibodies by exchanging murine constant region exons for human immunoglobulin constant region exons, thus generating chimeric antibodies with murine antibody-combining sites and human constant regions. An even more sophisticated technique in "humanizing" antibodies described in European Patent Application 0 239 400 exchanges also other fairly conserved regions, the so-called framework regions (FRs), within the murine variable regions for corresponding framework regions from human antibodies.

Such a humanized antibody should be even less immunogenic in man since the only parts derived from a murine antibody are those hypervariable regions which define a particular specificity for an antigen, the so-called complementarity determining regions (CDRs).

## Object of the invention

It is an object of this invention to provide monoclonal antibodies and antibody derivatives directed against HIV core protein p24 which recognize p24 expressed on the surface of V-infected macrophages and/or kill HIV-infected cells, in particular by mediating antibody-dependent cell-mediated cytotoxicity (ADCC). Some of these antibodies additionally inhibit the spread of infection from HIV-infected to non-infected cells and/or reduce the

EP 0 519 866 A1

amount of infectious HIV produced by macrophages and chronically infected lymphoid cells. The monoclonal antibodies of the invention may be murine antibodies or chimeric antibodies consisting of human constant regions and murine variable or hypervariable regions. Methods of manufacture of such antibodies, hybridoma or transfectoma cell lines secreting them and methods for production of the hybridoma or transfectoma cell lines are also encompassed by this invention. The invention further concerns recombinant DNA comprising an insert coding for the variable regions of antibodies against HIV core protein p24 having the mentioned properties, methods of manufacture of such recombinant DNA, and host cells transformed with such recombinant DNA suitable for the manufacture of recombinant antibodies.

The antibodies are especially useful for the prevention of the progression of AIDS, i.e. of the development from the asymptomatic infected stage to the actual outbreak of the disease, and for the treatment of AIDS, but can also be used for the diagnosis of HIV infection in an immunoassay.

Detailed description of the invention

The invention concerns monoclonal antibodies directed against HIV core protein p24 which recognize p24 expressed on the surface of HIV-infected macrophages. Preferred are monoclonal antibodies directed against HIV core protein p24 with the mentioned properties which kill HIV-infected cells, in particular by mediating antibody-dependent cell-mediated cytotoxicity (ADCC). Especially preferred are monoclonal anti-p24 antibodies which additionally inhibit the spread of infection from HIV-infected to non-infected cells, for example by preventing the production of HIV particles by HIV-infected cells, and/or reduce the amount of infectious HIV produced by macrophages and chronically infected lymphoid cells. Also especially preferred are monoclonal antibodies which further recognize p24 of diverse HIV strains, e.g. of HIV-1 and HIV-2 and variants thereof.

The invention further concerns derivatives of the antibodies of the invention which retain the specificity of the antibody from which they are derived.

The monoclonal antibodies of the invention may be murine antibodies or chimeric antibodies consisting of human constant regions and murine variable or hypervariable regions.

Murine monoclonal antibodies with the mentioned properties are for example MAb 25-57-1 and MAb 26-69-5. These antibodies and derivatives thereof are particularly preferred.

The invention especially concerns chimeric monoclonal antibodies consisting of human constant regions and murine variable regions of an antibody which is directed against HIV p24 with the mentioned properties, and derivatives thereof. This means that the chimeric MAbs of the invention have constant regions which are derived from a human antibody or have amino acid sequences which are homologous to constant region sequences of such a human Ab, and that the chimeric MAbs of the invention have variable regions which are derived from a murine antibody directed against HIV p24 or have amino acid sequences which are homologous to variable region sequences of such a murine MAb.

In particular, the invention concerns chimeric monoclonal antibodies consisting of human constant regions and murine variable regions which are derived from or which are homologous to the variable regions of the preferred murine monoclonal antibody 25-57-1 or of the preferred murine monoclonal antibody 26-69-5, respectively, and derivatives thereof.

Especially preferred is the chimeric monoclonal antibody designated MAb Ch25 which contains the variable regions of the murine monoclonal antibody 25-57-1, and derivatives thereof. Also especially preferred is the chimeric monoclonal antibody designated MAb Ch26 which contains the variable regions of the murine monoclonal antibody 26-69-5, and derivatives thereof.

The variable region of an antibody light chain consists of so-called framework regions (FRs), which are fairly conserved in antibodies with different specificities, and of hypervariable regions, also called complementarity determining regions (CDRs), which are typical for a particular specificity.

Preferred chimeric monoclonal antibodies of the invention and their derivatives are those wherein the light chain variable regions comprise a polypeptide of the formula

$$FR_1\text{-}CDR_{1L}\text{-}FR_2\text{-}CDR_{2L}\text{-}FR_3\text{-}CDR_{3L}\text{-}FR_4 \qquad (I)$$

wherein $FR_1$ is a polypeptide residue comprising 19 to 23 naturally occurring amino acids, $FR_2$ is a polypeptide residue comprising 13 to 17 naturally occurring amino acids, $FR_3$ is a polypeptide residue comprising 30 to 34 naturally occurring amino acids, $FR_4$ is a polypeptide residue comprising 7 to 11 naturally occurring amino acids, $CDR_{1L}$ is a polypeptide residue of the amino acid sequence 22 to 32 of SEQ ID NO:1, $CDR_{2L}$ is a polypeptide residue of the amino acid sequence 48 to 54 of SEQ ID NO:1, and $CDR_{3L}$ is a polypeptide residue of the amino acid sequence 87 to 95 of SEQ ID NO:1, and wherein the amino acid Cys may be in the oxidized state forming S-S-bridges. These particular complementarity determining regions are Arg-Ala-Ser-Glu-Asn-Ile-Tyr-Ser-Asn-Leu-Ala ($CDR_{1L}$), Ala-Ala-Thr-Asn-Leu-Ala-Asp ($CDR_{2L}$), and Gln-His-Phe-Trp-Ser-Thr-Pro-Trp-Thr ($CDR_{3L}$).

3

Especially preferred are chimeric monoclonal antibodies and derivatives thereof comprising light chain variable regions of formula I, wherein the polypeptide residues of the framework regions $FR_1$, $FR_2$, $FR_3$ and $FR_4$ are those preferably occurring in maminalian, especially murine or human, antibodies.

Most preferred are chimeric monoclonal antibodies and derivatives thereof according to the invention with light chain variable regions comprising a polypeptide of the amino acid sequence of SEQ ID NO:1, wherein optionally one or more, e.g. 1, 2, 3 or 4, single amino acids within the amino acid sequences 1 to 21 ($FR_1$), 33 to 47 ($FR_2$), 55 to 86 ($FR_3$), and/or 96 to 104 ($FR_4$) are replaced by other amino acids or deleted, and wherein the amino acid Cys may be in the oxidized state forming S-S-bridges, in particular the chimeric monoclonal antibodies and derivatives thereof with light chain variable regions comprising a polypeptide of the amino acid sequence 1 to 104 of SEQ ID NO:1, wherein the amino acid Cys may be in the oxidized state forming S-S-bridges. For example, a hydrophobic amino acid within the framework regions may be replaced by another amino acid, preferably also a hydrophobic amino acid, e.g. a homologous amino acid, replaced by two amino acids, or deleted. Likewise, a hydrophilic amino acid within the framework region may be replaced by another amino acid, two amino acids or deleted, whereby replacing amino acids preferably maintain the hydrogen bond structure of the corresponding framework region.

Likewise preferred chimeric monoclonal antibodies of the invention and their derivatives are those wherein the heavy chain variable regions comprise a polypeptide of the formula

$$FR_5\text{-}CDR_{1H}\text{-}FR_6\text{-}CDR_{2H}\text{-}FR_7\text{-}CDR_{3H}\text{-}FR_8 \qquad (II)$$

wherein $FR_5$ is a polypeptide residue comprising 25 to 29 naturally occuring amino acids, $FR_6$ is a polypeptide residue comprising 12 to 16 naturally occuring amino acids, $FR_7$ is a polypeptide residue comprising 30 to 34 naturally occuring amino acids, $FR_6$ is a polypeptide residue comprising 6 to 10 naturally occuring amino acids, $CDR_{1H}$ is a polypeptide residue of the amino acid sequence 28 to 32 of SEQ ID NO:2, $CDR_{2H}$ is a polypeptide residue of the amino acid sequence 47 to 63 of SEQ ID NO:2, and $CDR_{3H}$ is a polypeptide residue of the amino acid sequence 96 to 99 of SEQ ID NO:2, and wherein the amino acid Cys may be in the oxidized state forming S-S-bridges. These particular complementarity determining regions are Asp-Tyr-Ala-Met-His ($CDR_{1H}$), Ile-Ile-Arg-Thr-Tyr-Asn-Gly-Asn-Thr-Asn-Tyr-Asn-Gln-Lys-Gly ($CDR_{2H}$), and Asn-Val-Ala-Tyr($CDR_{3H}$).

Especially preferred are chimeric monoclonal antibodies and derivatives thereof comprising heavy chain variable regions of formula II, wherein the polypeptide residues of the framework regions $FR_5$, $FR_6$, $FR_7$ and $FR_6$ are those preferably occuring in mammalian, especially murine or human, antibodies.

Most preferred are chimeric monoclonal antibodies and derivatives thereof according to the invention with heavy chain variable regions comprising a polypeptide of the amino acid sequence of SEQ ID NO:2, wherein optionally one or more, e.g. 1, 2, 3 or 4, single amino acids within the amino acid sequences 1 to 27 ($FR_6$), 33 to 46 ($FR_6$), 64 to 95 ($FR_7$), and/or 100 to 107 ($FR_8$) are replaced by other amino acids or deleted, and wherein the amino acid Cys may be in the oxidized state forming S-S-bridges, in particular the chimeric monoclonal antibodies and derivatives thereof with heavy chain variable regions comprising a polypeptide of the amino acid sequence 1 to 107 of SEQ ID NO:2, wherein the amino acid Cys may be in the oxidized state forming S-S-bridges. For example, amino acids within the framework regions may be replaced by other amino acids or deleted as detailed above for the light chain.

Light chain variable regions and heavy chain variable regions may comprise an acyl residue at the N-terminal of SEQ ID NO:1 and SEQ ID NO:2, respectively, for example formyl or alkanoyl, e.g. palmitoyl, myristoyl or lower alkanoyl, such as acetyl or propionyl.

The invention preferentially concerns a chimeric monoclonal antibody and derivatives thereof with light chain variable regions of formula I with the preferred meaning, e.g. with the amino acid sequence given in SEQ ID NO:1, wherein the amino acid Cys may be in the oxidized state forming S-S-bridges, light chain human constant regions $\kappa$ or $\lambda$, in particular $\kappa$, heavy chain variable regions of formula II with the preferred meaning, e.g. with the amino acid sequence given in SEQ ID NO:2, wherein the amino acid Cys may be in the oxidized state forming S-S-bridges, and heavy chain human constant regions $\gamma 1$, $\gamma 2$, $\gamma 3$, or $\gamma 4$, in particular $\gamma 1$.

Also preferred chimeric monoclonal antibodies of the invention and their derivatives are those wherein the light chain variable regions comprise a polypeptide of the formula

$$FR_9\text{-}CDR_{4L}\text{-}FR_{10}\text{-}CDR_{5L}\text{-}FR_{11}\text{-}CDR_{6L}\text{-}FR_{12} \qquad (III)$$

wherein $FR_9$ is a polypeptide residue comprising 19 to 23 naturally occurring amino acids, $FR_{10}$ is a polypeptide residue comprising 13 to 17 naturally occurring amino acids, $FR_{11}$ is a polypeptide residue comprising 30 to 34 naturally occurring amino acids, $FR_{12}$ is a polypeptide residue comprising 7 to 11 naturally occurring amino acids, $CDR_{4L}$ is a polypeptide residue of the amino acid sequence 22 to 38 of SEQ ID NO:3, $CDR_{5L}$ is a polypeptide residue of the amino acid sequence 54 to 60 of SEQ ID NO:3, and $CDR_{6L}$ is a polypeptide residue of the amino acid sequence 93 to 101 of SEQ ID NO:3, and wherein the amino acid Cys may be in the oxidized state forming S-S-bridges. These particular complementarity determining regions are Lys-Ser-Ser-Gln-Ser-Leu-Leu-Tyr-Ser-Ser-Asn-Gln-Lys-Asn-Tyr-Leu-Ala ($CDR_{4L}$), Trp-Ala-Ser-Thr-Arg-Glu-Ser (CDR$_{5L}$), and Gln-Gln-Tyr-Tyr-Ser-Tyr-Pro-Trp-Thr (CDR$_{5L}$).

Especially preferred are chimeric monoclonal antibodies and derivatives thereof comprising light chain variable regions of formula III, wherein the polypeptide residues of the framework regions FR$_9$, FR$_{10}$, FR$_{11}$ and FR$_{12}$ are those preferably occurring in mammalian, especially murine or human, antibodies.

Most preferred are chimeric monoclonal antibodies and derivatives thereof according to the invention with light chain variable regions comprising a polypeptide of the amino acid sequence of SEQ ID NO:3, wherein optionally one or more, e.g. 1, 2, 3 or 4, single amino acids within the amino acid sequences 1 to 21 (FR$_9$), 39 to 53 (FR$_{10}$), 61 to 92 (FR$_{11}$), and/or 102 to 110 (FR$_{12}$) are replaced by other amino acids or deleted, and wherein the amino acid Cys may be in the oxidized state forming S-S-bridges, in particular the chimeric monoclonal antibodies and derivatives thereof with light chain variable regions comprising a polypeptide of the amino acid sequence 1 to 110 of SEQ ID NO:3, wherein the amino acid Cys may be in the oxidized state forming S-S-bridges. For example, a hydrophobic amino acid within the framework regions may be replaced by another amino acid, preferably also a hydrophobic amino acid, e.g. a homologous amino acid, replaced by two amino acids, or deleted. Likewise, a hydrophic amino acid within the framework region may be replaced by another amino acid, two amino acids or deleted, whereby replacing amino acids preferably maintain the hydrogen bond structure of the corresponding framework region.

Likewise preferred chimeric monoclonal antibodies of the invention and their derivatives are those wherein the heavy chain variable regions comprise a polypeptide of the formula

$$FR_{13}\text{-}CDR_{4H}\text{-}FR_{14}\text{-}CDR_{5H}\text{-}FR_{15}\text{-}CDR_{6H}\text{-}FR_{16} \qquad (IV)$$

wherein FR$_{13}$ is a polypeptide residue comprising 25 to 29 naturally occurring amino acids, FR$_{14}$ is a polypeptide residue comprising 12 to 16 naturally occurring amino acids, FR$_{15}$ is a polypeptide residue comprising 30 to 34 naturally occurring amino acids, FR$_{16}$ is a polypeptide residue comprising 6 to 10 naturally occurring amino acids, CDR$_{4H}$ is a polypeptide residue of the amino acid sequence 28 to 32 of SEQ ID NO:4, CDR$_{5H}$ is a polypeptide residue of the amino acid sequence 47 to 63 of SEQ ID NO:4, and CDR$_{6H}$ is a polypeptide residue of the amino acid sequence 96 to 109 of SEQ ID NO:4, and wherein the amino acid Cys may be in the oxidized state forming S-S-bridges. These particular complementarity determining regions are Met-Tyr-Trp-Leu-Glu (CDR$_{4H}$), Glu-Ile-Ser-Pro-Gly-Thr-Phe-Thr-Thr-Asn-Tyr-Asn-Glu-Lys-Phe-L ys-Ala (CDR$_{5H}$), and Phe-Ser-His-Tyr-Ser-Gly-Asn-Asn-Tyr-Asp-Tyr-Phe-Asp-Tyr (CDR$_{5H}$).

Especially preferred are chimeric monoclonal antibodies and derivatives thereof comprising heavy chain variable regions of formula IV, wherein the polypeptide residues of the framework regions FR$_{13}$, FR$_{14}$, FR$_{15}$ and FR$_{16}$ are those preferably occurring in mammalian, especially murine or human, antibodies.

Most preferred are chimeric monoclonal antibodies and derivatives thereof according to the invention with heavy chain variable regions comprising a polypeptide of the amino acid sequence of SEQ ID NO:4, wherein optionally one or more, e.g. 1, 2, 3 or 4, single amino acids within the amino acid sequences 1 to 27 (FR$_{13}$), 33 to 46 (FR$_{14}$), 64 to 95 (FR$_{15}$), and/or 110 to 117 (FR$_{16}$) are replaced by other amino acids or deleted, and wherein the amino acid Cys may be in the oxidized state forming S-S-bridges, in particular the chimeric monoclonal antibodies and derivatives thereof with heavy chain variable regions comprising a polypeptide of the amino acid sequence 1 to 117 of SEQ ID NO:4, wherein the amino acid Cys may be in the oxidized state forming S-S-bridges. For example, amino acids within the framework regions may be replaced by other amino acids or deleted as detailed above for the light chain.

Light chain variable regions and heavy chain variable regions may comprise an acyl residue at the N-terminal of SEQ ID NO:3 and SEQ ID NO:4, respectively, for example formyl or alkanoyl, e.g. palmitoyl, myristoyl or lower alkanoyl, such as acetyl or propionyl.

The invention preferentially concerns a chimeric monoclonal antibody and derivatives thereof with light chain variable regions of formula III with the preferred meaning, e.g. with the amino acid sequence given in SEQ ID NO:3, wherein the amino acid Cys may be in the oxidized state forming S-S-bridges, light chain human constant regions $\kappa$ or $\lambda$, in particular $\kappa$, heavy chain variable regions of formula IV with the preferred meaning, e.g. with the amino acid sequence given in SEQ ID NO:4, wherein the amino acid Cys may be in the oxidized state forming S-S-bridges, and heavy chain human constant regions $\gamma1$, $\gamma2$, $\gamma3$ or $\gamma4$, in particular $\gamma1$.

The class of an antibody (immunoglobulin, Ig) molecule is defined by the heavy chain regions. A chimeric monoclonal antibody of the invention may be of any immunoglobulin class, i.e. IgA, IgD, IgE, IgG or IgM. Since different isotypes of antibodies may have different immune-regulatory action, the MAbs can be chosen accordingly. A preferential chimeric monoclonal antibody according to the invention is an immunoglobulin of class G which comprises light chain human constant regions $\kappa$ or $\lambda$, especially human constant regions $\kappa$, and heavy chain human constant regions $\gamma1$, $\gamma2$, $\gamma3$ or $\gamma4$, especially human constant regions $\gamma1$.

Derivatives of a monoclonal antibody of the invention retain the specificity of the antibody from which they are derived, i.e. they retain the characteristic binding pattern of the parent antibody. Examples of such derivatives are chimeric monoclonal antibody fragments, conjugates of the antibody or of an antibody fragment with an enzyme, with a fluorescent marker, with a chemiluminescent marker, with a metal chelate, with avidin, with biotin or the like, or radioactively labelled antibodies or antibody fragments.

Antibody fragments of the invention are for example the univalent fragments Fab or Fab' or the divalent fragment F(ab')$_2$. A fragment in a conjugate of the invention may also be a fragment Fv.

Enzymes used for antibody conjugates of the invention are, for example, horseradish peroxidase, alkaline phosphatase, β-D-galactosidase, glucose oxidase, glucoamylase, carbonic anhydrase, acetylcholinesterase, lysozyme, malate dehydrogenase or glucose-6-phosphate dehydrogenase.

Fluorescent markers conjugated with antibodies or fragments of the invention can be fluorescein, fluorochrome, rhodamine, and the like.

Chemiluminescence markers are e.g. acridinium esters of luminol.

In such conjugates, the chimeric antibody or fragment is bound to the conjugation partner directly or by way of a spacer or linker group.

Examples of metal chelates are ethylenediaminetetraacetic acid (EDTA), diethylene-triaminepentaacetic acid (DPTA), 1,4,8,11-tetraazatetradecane, 1,4,8,11-tetraazatetradecane-1,4,8,11-tetraacetic acid, 1-oxa-4,7,12,15-tetraazaheptadecane-4,7,12,15-tetraacetic acid, or the like.

Radioactively labelled antibodies or fragments of the invention contain e.g. radioactive iodine ($^{123}$I, $^{125}$I, $^{131}$I), tritium ($^3$H), carbon ($^{14}$C), sulfur ($^{35}$S), yttrium ($^{90}$Y), technetium ($^{99m}$Tc), or the like.

The MAbs of the invention are tested
. for their specificity to natural antigen HIV p24 or cell-associated viral antigen in T-cells, T-cell lines, monocytes or macrophages, for example in an immunoassay such as an immuno-staining assay or a binding enzyme immunoassay;
. for their ability to mediate ADCC (antibody-dependent cell-mediated cytotoxicity) in an assay determining the release of $^{51}$chromium from HIV-infected target cells;
. for their effect on HIV-infected human macrophages or H9 cells by determining reverse transcriptase production by infected cells; and/or
. for their capability to inhibit the spread of infection from infected H9 cells to uninfected cells in coculture experiments.

The monoclonal antibodies and derivatives thereof according to the invention are prepared by processes that are known per se, characterized in that mammalian cells as defined further below producing such monoclonal antibodies are multiplied in vitro or in vivo and, when required, the obtained monoclonal antibodies are isolated and/or converted into derivatives thereof.

Multiplication in vitro is carried out in suitable culture media, which are the customary standard culture media, for example Dulbecco's Modified Eagle Medium (DMEM) or RPMI 1640 medium, optionally replenished by a mammalian serum, e.g. fetal calf serum, or trace elements and growth sustaining supplements, e.g feeder cells such as normal mouse peritoneal cells, spleen cells, bone marrow macrophages, 2-aminoethanol, insulin, transferrin, low density lipoprotein, oleic acid, or the like.

In vitro production provides relatively pure antibody preparations and allows scale-up to give large amounts of the desired antibodies. Techniques for mammalian cell cultivation under tissue culture conditions are known in the art and include homogeneous suspension culture, e.g. in an airlift reactor or in a continuous stirrer reactor, or immobilized or entrapped cell culture, e.g. in hollow fibres, microcapsules, on agarose microbeads or ceramic cartridges.

Large quantities of the desired monoclonal antibodies can also be obtained by multiplying the cells in vivo. For this purpose, cells of a continuous cell line producing the desired antibodies are injected into histocompatible mammals to cause growth of antibody-producing tumours. Optionally, the animals are primed with a hydrocarbon, especially mineral oils such as pristane (tetramethyl pentadecane), prior to the injection. After one to three weeks, the antibodies are isolated from the body fluids of those mammals. For example, cells derived from hybridoma cell line Sp2/0 that produce the desired antibodies are injected intraperitoneally into Balb/c mice optionally pre-treated with pristane, and, after one to two weeks, ascitic fluid is taken from the animals.

The cell culture supernatants are screened for the desired monoclonal antibodies, preferentially with an enzyme immunoassay, e.g. a sandwich assay or a dot-assay, or a radioimmunoassay. For example, antigen is dotted on nitrocellulose discs, incubated with culture fluids of growing hybridomas, then alkaline phosphatase labelled anti-mouse IgG and a substrate solution. The presence of the desired antibodies is indicated by development of a coloured dot.

For isolation of the monoclonal antibodies, the immunoglobulins in the culture supernatants may be concentrated, e.g. by precipitation with ammonium sulphate, dialysis against hygroscopic material such as PEG, filtration through selective membranes, or the like. If necessary and/or desired, the antibodies are purified by the customary chromatography methods, for example gel filtration, ion-exchange chromatography, chromatography over DEAE-cellulose or (immuno-)affinity chromatography. Preferably, the monoclonal antibodies are isolated from cell supernatants containing them by affinity chromatography, for example with Protein A, and/or ion-exchange chromatography.

Fragments of the monoclonal antibodies, for example Fab, Fab'or F(ab')$_2$ fragments, can be obtained from the antibodies prepared as described above by methods known per se, e.g. by digestion with enzymes such as papain or pepsin and/or cleavage of disulfide bonds by chemical reduction.

Conjugates of antibodies or antibody fragments of the invention with the mentioned enzymes are prepared e.g. by reacting an antibody or fragment prepared as described above with the enzyme in the presence of a coupling agent, e.g. glutaraldehyde, periodate, N,N'-o-phenylenedimaleimide, N-(m-maleimidobenzoyloxy)-succinimide, N-(3-[2'-pyridyldithio]-propionoxy)-succinimide, N-ethyl-N'-(3-dimethylamino-propyl)-carbodiimide or the like. Conjugates with biotin are prepared e.g. by reacting antibodies or antibody fragments with an activated ester of biotin such as the biotin N-hydroxysuccinimide ester. Conjugates with fluorescent or chemiluminescent markers are prepared in the presence of a coupling agent, e.g. those listed above, or by reaction with an isothiocyanate, preferably fluorescein-isothiocyanate.

Monoclonal antibodies or antibody fragments radioactively labelled with iodine ($^{123}$I, $^{125}$I, $^{131}$I) are obtained from the antibodies or fragments of the invention by iodination known per se, for example with radioactive sodium or potassium iodide and a chemical oxidizing agent, such as sodium hypochlorite, chloramine T or the like, or an enzymatic oxidizing agent, such as lactoperoxidase, or glucose oxidase and glucose. Antibodies or antibody fragments according to the invention are coupled to yttrium ($^{90}$Y) for example by diethylenetriamine-pentaacetic acid (DPTA)-chelation. Technetium-99m labelled antibodies or antibody fragments are prepared by ligand exchange processes, for example by reducing pertechnate ($TcO_4^-$) with stannous ion solution, chelating the reduced technetium onto a Sephadex column and applying the antibodies or fragments to this column, or by direct labelling techniques, e.g. by incubating pertechnate, a reducing agent such as $SnCl_2$, a buffer solution such as sodium-potassium phthalate solution, and the antibodies or antibody fragments.

The invention also concerns recombinant DNAs comprising an insert coding for a light chain murine variable region and/or for a heavy chain murine variable region of anti-p24 monoclonal antibodies as described hereinbefore. By definition such DNAs comprise coding single stranded DNAs, double stranded DNAs consisting of said coding DNAs and of complementary DNAs thereto, or these complementary (single stranded) DNAs themselves.

In particular the invention concerns a recombinant DNA comprising an insert coding for a light chain murine variable region, which originates from genomic DNA or mRNA of the hybridoma cell line 25-57-1 or which is homologous to genomic DNA of said cell line and codes for an amino acid sequence homologous to the light chain variable region of monoclonal antibody 25-57-1. The hybridoma cell line 25-57-1 was generated by fusing myeloma cells and B lymphocytes of mice immunized with p25. The cell line 25-57-1 produces the murine antibody MAb 25-57-1.

Preferred is a recombinant DNA comprising an insert coding for the polypeptide of formula I, wherein $FR_1$, $FR_2$, $FR_3$, $FR_4$, $CDR_{1L}$, $CDR_{2L}$, and $CDR_{3L}$ have the meanings as mentioned hereinbefore, optionally further containing introns. Especially preferred is a recombinant DNA coding for the polypeptide of formula I comprising inserts coding for murine or human framework regions $FR_1$, $FR_2$, $FR_3$ and $FR_4$, and inserts coding for complementarity determining regions of the DNA sequence 70 to 102 ($CDR_{1L}$), the DNA sequence 148 to 168 ($CDR_{2L}$), and the DNA sequence 265 to 291 ($CDR_{3L}$) of SEQ ID NO:1. Most preferred is a DNA comprising an insert of the DNA sequence 7 to 318 of SEQ ID NO:1, wherein optionally one or more, e.g. 1 to 10, nucleotides are replaced by other nucleotides, in particular a DNA comprising an insert of the DNA sequence 7 to 318 of SEQ ID NO:1. In a DNA wherein nucleotides of the sequence given in SEQ ID NO:1 are replaced by other nucleotides, such replacement is preferred when it does not alter the amino acid sequence of the complementarity determining regions (CDRs) coded for. This means that such replacement of nucleotides may occur in the inserts coding for the framework regions (FRs) or in a position where it does not alter the encoded amino acid due to the degeneracy of the triplet codons.

Likewise the invention concerns a recombinant DNA comprising an insert coding for a heavy chain murine variable region, which originates from genomic DNA or mRNA of the hybridoma cell line 25-57-1 or which is homologous to genomic DNA of said cell line and codes for an amino acid sequence homologous to the heavy chain variable region of monoclonal antibody 25-57-1.

Preferred is a recombinant DNA comprising an insert coding for the polypeptide of formula II, wherein $FR_5$, $FR_6$, $FR_7$, $FR_8$, $CDR_{1H}$, $CDR_{2H}$, and $CDR_{3H}$ have the meanings as mentioned hereinbefore, optionally further containing introns. Especially preferred is a recombinant DNA coding for the polypeptide of formula II comprising inserts coding for murine or human framework regions $FR_6$, $FR_6$, $FR_7$ and $FR_6$, and inserts coding for complementarity determining regions of the DNA sequence 90 to 104 ($CDR_{1H}$), the DNA sequence 147 to 197 ($CDR_{2H}$), and the DNA sequence 294 to 305 ($CDR_{3H}$) of SEQ ID NO:2. Most preferred is a DNA comprising an insert of the DNA sequence 9 to 329 of SEQ ID NO:2, wherein optionally one or more, e.g. 1 to 10, nucleotides are replaced by other nucleotides, in a DNA comprising an insert of the DNA sequence 9 to 329 of SEQ ID NO:2. In a DNA wherein nucleotides of the sequence given in SEQ ID NO:2 are replaced by other nucleotides, such replacement is preferred when it does not alter the amino acid sequence of the complementarity determining regions (CDRs) coded for, as is described above for DNA coding for the light chain variable region.

In particular the invention concerns a recombinant DNA comprising an insert coding for a light chain murine variable region, which originates from genomic DNA of the hybridoma cell line 26-69-5 or which is homologous to genomic DNA or mRNA of said cell line and codes for an amino acid sequence homologous to the light chain variable region of monoclonal antibody 26-69-5. The hybridoma cell line 26-69-5 was generated by fusing myeloma cells and B lymphocytes of mice immunized with p25. The cell line 26-69-5 produces the murine antibody MAb 26-69-5.

Preferred is a recombinant DNA comprising an insert coding for the polypeptide of formula III, wherein $FR_9$, $FR_{10}$, $FR_{11}$, $FR_{12}$, $CDR_{4L}$, $CDR_{5L}$, and $CDR_{6L}$ have the meanings as mentioned hereinbefore, optionally further containing introns. Especially preferred is a recombinant DNA coding for the polypeptide of formula III comprising inserts coding for murine or human framework regions $FR_9$, $FR_{10}$, $FR_{11}$ and $FR_{12}$, and inserts coding for complementarity determining regions of the DNA sequence 70 to 120 ($CDR_{4L}$), the DNA sequence 166 to 186 ($CDR_{5L}$), and the DNA sequence 283 to 309 ($CDR_{5L}$) of SEQ ID NO:3. Most preferred is a DNA comprising an insert of the DNA sequence 7 to 336 of SEQ ID NO:3, wherein optionally one or more, e.g. 1 to 10, nucleotides are replaced by other nucleotides, in particular a DNA comprising an insert of the DNA sequence 7 to 336 of SEQ ID NO:3. In a DNA wherein nucleotides of the sequence given in SEQ ID NO:3 are replaced by other nucleotides, such replacement is preferred when it does not alter the amino acid sequence of the complementarity determining regions (CDRs) coded for. This means that such replacement of nucleotides may occur in the inserts coding for the framework regions (FRs) or in a position where it does not alter the amino acid coded for due to the degeneracy of the triplet codons.

Likewise the invention concerns a recombinant DNA comprising an insert coding for a heavy chain murine variable region, which originates from genomic DNA or mRNA of the hybridoma cell line 26-69-5 or which is homologous to genomic DNA of said cell line and codes for an amino acid sequence homologous to the heavy chain variable region of monoclonal antibody 26-69-5.

Preferred is a recombinant DNA comprising an insert coding for the polypeptide of formula IV, wherein $FR_{13}$, $FR_{14}$, $FR_{15}$, $FR_{16}$, $CDR_{4H}$, $CDR_{5H}$, and $CDR_{6H}$ have the meanings as mentioned hereinbefore, optionally further containing introns. Especially preferred is a recombinant DNA coding for the polypeptide of formula IV comprising inserts coding for murine or human framework regions $FR_{13}$, $FR_{14}$, $FR_{15}$ and $FR_{16}$, and inserts coding for complementarity determining regions of the DNA sequence 90 to 104 ($CDR_{4H}$), the DNA sequence 147 to 197 ($CDR_{5H}$), and the DNA sequence 294 to 335 ($CDR_{5H}$) of SEQ ID NO:4. Most preferred is a DNA comprising an insert of the DNA sequence 9 to 359 of SEQ ID NO:4, wherein optionally one or more, e.g. 1 to 10, nucleotides are replaced by other nucleotides, in particular a DNA comprising an insert of the DNA sequence 9 to 359 of SEQ ID NO:4. In a DNA wherein nucleotides of the sequence given in SEQ ID NO:4 are replaced by other nucleotides, such replacement is preferred when it does not alter the amino acid sequence of the complementarity determining regions (CDRs) coded for, as is described above for DNA coding for the light chain variable region.

For the assembly of complete tetrameric immunoglobulin molecules and the expression of active antibodies, the recombinant DNA inserts coding for light and heavy chain variable regions are fused with the corresponding DNAs coding for light and heavy chain constant regions, then transferred into appropriate host cells, for example after incorporation into hybrid vectors.

The invention therefore also concerns recombinant DNAs comprising an insert coding for a light chain murine variable region of an anti-p24 antibody with the described properties, fused to a human constant region κ or λ. Preferred is a recombinant DNA coding for a preferred murine variable region as described hereinbefore fused to a human constant region κ.

Likewise the invention concerns recombinant DNAs comprising an insert coding for a heavy chain murine variable region of an anti-p24 antibody with the described properties, fused to a human constant region γ, for example γ1, γ2, γ3 or γ4. Preferred is a recombinant DNA coding for a preferred murine variable region as described hereinbefore fused to a human constant region γ1.

The invention further concerns recombinant DNAs coding for fragments of chimeric monoclonal antibodies as defined hereinbefore, e.g. DNA coding for Fab, Fab'or Fv fragments of such antibodies, and for conjugates of antibodies or fragments as defined hereinbefore, e.g. for fusion proteins comprising a light or heavy chain of such antibody fused to an enzyme.

Furthermore the invention concerns a recombinant DNA which is a hybrid vector comprising an insert coding for a chimeric murine/human light chain as described hereinbefore and/or an insert coding for a chimeric murine/human heavy chain as described hereinbefore, an origin of replication or an autonomously replicating sequence, one or more dominant marker sequences and, optionally, expression control sequences, signal sequences and functional restriction sites.

Vectors typically perform two functions in collaboration with compatible host cells. One function is to facilitate the cloning of the nucleic acid that encodes the chimeric immunoglobulin chains, i.e. to produce usable quantities of the nucleic acid (cloning vectors). The other function is to provide for replication and expression of the chimeric gene constructs in a suitable host, either by maintenance as an extrachromosomal element or by integration into the host chromosome (expression vectors). A cloning vector comprises the chimeric gene constructs as described above, an origin of replication or an autonomously replicating sequence, dominant marker sequences and, optionally, signal sequences and functional restriction sites. An expression vector additionally comprises expression control sequences essential for the transcription and translation of the chimeric genes.

An origin of replication or an autonomously replicating sequence is provided either by construction of the vector to include an exogeneous origin such as derived from Simian virus 40 (SV 40) or another viral source, or by the host cell chromosomal mechanisms.

The markers allow for selection of host cells which contain the vector. Selection markers include genes which confer resistance to heavy metals such as copper or to antibiotics such as tetracycline, ampicillin, geneticin (G-418), neomycin, kanamycin or hygromycin, or genes which complement a genetic lesion of the host cell such as the absence of thymidin kinase, hypoxanthine phosphoryl transferase, dihydrofolate reductase or the like.

Signal sequences may be, for example, presequences or secretory leaders directing the secretion of the antibody, splice signals, or the like.

As expression control sequences, the vector DNA comprises a promoter, sequences necessary for the initiation and termination of transcription and for stabilizing the mRNA and, optionally, enhancers and further regulatory sequences. A wide variety of promoting sequences may be employed, depending on the nature of the host cell. Promoters suitable for mammalian alian host cells are obtained from viruses such as Simian virus 40 (SV 40), Rous sarcoma virus (RSV), adenovirus 2, bovine papilloma virus (BPV), papovavirus BK mutant (BKV), or mouse or human cytomegalovirus (CMV). Alternatively, the vectors may comprise promoters from mammalian expression products, such as actin, collagen, myosin etc., or the native promoter and control sequences which are normally associated with the immunoglobulin gene sequences. Sequences necessary for the initiation and termination of transcription and for stabilizing the mRNA are commonly available from the noncoding 5' regions and 3' regions, respectively, of viral and eukaryotic cDNAs, e.g. from the expression host. Enhancers are transcription-stimulating DNA sequences of viral origin, e.g. derived from Simian virus, polyoma virus, bovine papilloma virus or Moloney sarcoma virus, or of genomic, especially murine, origin.

The various DNA segments of the vector DNA are operationally linked, i.e. they are contiguous and placed into a functional relationship with each other.

Preferred vectors are suitable for mammalian hosts and are based on viral replication systems. Particularly preferred are vectors comprising Simian virus promoters, e.g. pSVgpt or pSVneo, further comprising an enhancer, e.g. an enhancer normally associated with the immunoglobulin gene sequences, in particular the mouse Ig H or L chain enhancer.

The chimeric gene constructs for the light chain and for the heavy chain are sequentially or simultaneously transferred into the host cells with the help of two vectors. Alternatively, both heavy and light chains are cloned into the same hybrid vector and incorporated in a one step-procedure as a single construct into the host cells. A third alternative utilises co-transfection of unlinked DNAs fragments.

The recombinant DNAs coding for the desired chimeric monoclonal antibodies can be prepared, for example, by culturing a transformed host cell.

In particular, such DNAs can be prepared by

a) isolating murine DNAs from a suitable hybridoma cell line and selecting the desired DNAs coding for the variable regions of antibodies with the desired specificity using DNA probes,

b) isolating human DNAs from a genomic library and selecting the desired DNAs coding for the constant regions of antibodies using DNA probes,

c) constructing chimeric mouse/human genes by incorporating the DNA of step a) and b) into appropriate hybrid vectors,

d) transferring the obtained hybrid vectors into a recipient host cell or retrieving the DNA coding for the chimeric mouse/human genes and transferring the unlinked DNA into a recipient host cell,

e) selecting and culturing the transformed host cell, and

f) optionally isolating the desired DNA.

The DNA according to step a) of the process described above can be obtained by isolation of genomic DNA or by preparation of cDNA from isolated mRNA. Genomic DNA from hybridoma cells is isolated by methods known in the art which include steps for disruption of the cells, e.g. by lysis in presence of detergents like Triton™, extracting the DNA, e.g. by treatment with phenol and CHCl₃/isoamyl alcohol, and precipitation of DNA. The DNA is fragmented, conveniently by one or more restriction endonucleases, e.g. XbaI, BglII, EcoRI, HindIII, BamHI, the resulting fragments are replicated on a suitable carrier, e.g. nitrocellulose membranes, and screened with a DNA probe as described in more detail hereinbelow for the presence of the DNA sequences coding for the polypeptide sequence of interest, in particular for the presence of the rearranged H- and L-chain Ig gene loci. By this procedure DNA fragments are found that contain inserts with heavy chain V, D and J regions and light chain V and J regions, respectively, together with a leader sequence and introns, if any. cDNA from hybridoma cells is likewise prepared by methods known in the art, e.g. by extracting total cellular RNA, isolating mRNA by a suitable chromatographic method, e.g. chromatography on oligo(dT)-cellulose, synthesizing cDNA with a mixture of deoxynucleotide triphosphate and reverse transcriptase in the presence of oligonucleotide primers complementary to suitable regions in the murine immunoglobulin heavy and light chain constant genes, and isolating the cDNA. As a tool simplifying DNA isolation, the desired genomic DNA or cDNA may be amplified using polymerase chain reaction (PCR) technology. PCR involves repeated rounds of extension from two primers specific for DNA regions at each end of the gene segment. Preferably, cDNA transcripts of total mRNA from the suitable hybridoma cell line is treated in a heating/cooling cycle with Taq DNA polymerase in the presence of primers tailored to hybridize to Ig H and L chain variable regions, respectively.

Genomic human DNA according to step b) of the process described above is isolated from suitable human tissue, preferably from human placenta or human foetal liver cells, according to methods known in the art. A genomic DNA library is constructed therefrom by limited digestion with suitable restriction endonucleases, e.g. HaeIII and AluI, and incorporation into λ Charon phage, e.g. λ Charon 4a, following established procedures. The genomic DNA library is replicated, e.g. on nitrocellulose membranes, and screened with a DNA probe as described below for the DNA sequences of interest. The desired DNA may be amplified using PCR technology.

The DNA probe for the mouse variable regions or the human constant regions may be a synthetic DNA, a cDNA derived from mRNA coding for the desired immunoglobulin or a genomic DNA or DNA fragment of known nucleotide sequence. As probes for the detection and/or amplification of the rearranged Ig gene loci of the variable regions of L-/H-chains, DNA fragments of known nucleotide sequences of adjacent conserved variable or constant regions are selected which constitute the Ig loci of the L-/H-chain in the mammal from which the DNA is derived, e.g. Balb/c mice. The possible utilization of murine DNA probes for the detection of human DNA sequences is based on sequence homologies between the murine and human DNAs. The DNA probe is synthesized or isolated from suitable tissue of an appropriate mammal, e.g. Balb/c mouse liver, and purified by standard methods. If required, the probe DNA is labelled, e.g. radioactively labelled by the well-known nick-translation technique, then hybridized with the human DNA library in buffer and salt solutions containing adjuncts, e.g. calcium chelators, viscosity regulating compounds, proteins, non-specific DNA and the like, at temperatures favoring selective hybridization.

Once a fragment has been identified which contains the desired DNA sequence, this fragment may be further manipulated to remove nonessential DNA, modified at one or both termini, and treated to remove all or a portion of intervening sequences, or the like.

The joining of the various DNA fragments in order to produce chimeric genes is performed in accordance with conventional techniques, for example, by blunt- or staggered-end ligation, restriction enzyme digestion to provide for appropriate cohesive termini, filling in cohesive ends as appropriate, alkaline phosphatase treatment to avoid undesirable joining, and ligation with appropriate ligases.

The transfer of the recombinant DNAs, e.g. the transfer of hybrid vectors, and the selection of transformed cells is described below.

The invention further concerns continuous cell lines. In one embodiment of the invention, such cell lines are hybridoma cell lines secreting anti-p24 monoclonal antibodies of the invention with the desired specificity.

In particular, the invention concerns hybridoma cell lines which are hybrids of myeloma cells and B lymphocytes of a mammal al immunized with HIV p25, optionally mixed with an adjuvant. Especially preferred are the hybridoma cell lines with the designation 25-57-1 and 26-69-5, respectively, which were deposited at the European Collection of Animal Cell Cultures (ECACC), PHLS Centre for Applied Microbiology & Research, Porton Down, Salisbury, Wilts. SP4 OJG, U.K., under the numbers ECACC 91040320 and ECACC 91040321, respectively, on April 3, 1991.

The invention also concerns continuous cell lines which are transfectoma cell lines secreting chimeric monoclonal antibodies consisting of murine variable regions and human constant regions according to the invention. By definition, transfectoma cell lines are transformed host cells such as immortalized mammalian cell lines, e.g. lymphoma, myeloma, hybridoma, trioma or quadroma cell lines, transformed with the recombinant DNAs described above, namely with a DNA encoding the light chain and/or a DNA encoding the heavy chain of the desired chimeric monoclonal antibody.

The host cells of the present invention have to be capable of culture in vitro and have to be of higher eukaryotic origin to provide a suitable environment for the production of active antibodies, since the biosynthesis of functional tetrameric antibody molecules requires correct nascent polypeptide chain folding, glycosylation, and assembly.

Examples of suitable host cells according to the invention are mammalian cells, e.g. COS-7 cells, Bowes melanoma cells, chinese hamster ovary (CHO) cells, embryonic lung cells L-132, and in particular mammalian cells of lymphoid origin, such as lymphoma, myeloma, hybridoma, trioma or quadroma cells, for example PAI, Sp2/0 or X63-Ag8.653 cells. Preferred are cells of the cell line Sp2/0, which is a well-characterized, Ig non-secreting mouse cell line derived from the fusion of mouse spleen cells with the myeloma X63-Ag8.

These host cells are transfected with the chimeric L-chain gene construct alone, with the chimeric H-chain gene construct alone, or with both, either sequentially or simultaneously transferred with the help of two separate vectors or in a one-step procedure by using a double-construct (L-chain/ H-chain) vector as indicated hereinbefore. In the alternative, unlinked chimeric gene constructs may be transfected into the host cells either sequentially or simultaneously.

Preferred are host cells transfected with both gene constructs secreting chimeric monoclonal anti-p24 antibodies as described hereinbefore, for example cells of the cell lines with the designation Ch25 and Ch26, respectively, which were deposited at the European Collection of Animal Cell Cultures (ECACC), PHLS Centre for Applied Microbiology & Research, Porton Down, Salisbury, Wilts. SP4 OJG, U.K., under the numbers ECACC 91052905 and ECACC 91052906, respectively, on May 29, 1991. Further examples of host cells of the invention are cells transfected with similar recombinant plasmids which contain alternative orientations of the H- and L-chain gene constructs, incorporating additional DNA elements to facilitate high levels of expression of the chimeric monoclonal antibodies.

The continuous cell lines of the invention are genetically stable, secrete monoclonal antibodies of the invention of constant specificity and can be activated from deep-frozen cultures by thawing and recloning.

The invention also concerns a process for the preparation of hybridoma cell lines secreting the monoclonal antibodies of the invention wherein a suitable mammal al is immunized with p25, optionally mixed with an adjuvant, antibody-producing cells of this mammal are fused with cells of a continuous cell line, the hybrid cells obtained in the fusion are cloned, and cell clones secreting the desired antibodies are selected.

The antigen is used to immunize suitable mammals which recognize it as a foreign molecule, for example mice, rats, rabbits, donkeys, goats, sheep, horses, pigs or chimpanzees, especially mice or rats, preferentially mice. Particularly preferred are Balb/c mice.

The immunogen may be mixed with adjuvants, i.e. agents that will further increase the immune response, for the immunization procedure. Possible adjuvants are Freund's complete adjuvant (emulsion of mineral oil, water, and mycobacterial extracts), Freund's incomplete adjuvant (emulsion of water and oil only), mineral gels, e.g. aluminium hydroxide gels, surface active substances such as lysolecithin, BCG (Bacillus Calmette-Guerin), polyanions, peptides such as N-acetylmuramyl-L-alanyl-D-isoglutamine, IL-2 or rat IFNγ, etc..

The routes of immunization include, among others, intradermal, subcutaneous, intramuscular, intraperitoneal, intravascular and intracranial injections. Since high antibody titers are desired, a series of injections is commonly given. The immunization is for example performed by injecting the antigen, optionally mixed with incomplete or complete Freund's adjuvant, three to eight times parenterally, e.g. intraperitoneally and/or subcutaneously, in amounts of around 10-150 μg into RA 25 rats, Balb/c mice or Biozzi mice at intervals of 1-3 weeks, followed by a booster injection of about 5-50 μg 1-5 days prior to sacrificing the animals.

Antibody-producing cells of the immunized mammals, preferably lymphoid cells such as spleen lymphocytes, taken for example 1-5 days after the final booster injection, are fused with the cells of a continuous cell line, i.e. a continuously replicating cell clone which confers this replication ability to the hybrid cells resulting from the fusion. An example for such a cell line is a tumor cell line (myeloma) which does not itself produce immunoglobulins or fragments thereof but has the potential to produce and secrete large amounts of antibody, and which carries a genetic marker so that the hybrid cells can be selected against non-fused parent cells. Several suitable myeloma cell lines are known in the art. Preferred are myeloma cell lines lacking the enzyme hypoxanthine guanine phosphoribosyl transferase (HGPRT) or the enzyme thymidine kinase (TK), which therefore do not survive in a selective culture medium containing hypoxanthine, aminopterin and thymidine (HAT medium). Particularly preferred are myeloma cells and derived cell lines that do not survive in HAT medium and do not secrete immunoglobulins or fragments thereof, such as the cell lines PAI, X63-Ag8.653 or Sp2/O-Ag14.

The fusion is performed in the presence of a fusion promoter, for example Sendai virus or other paramyxo viruses, optionally in UV-inactivated form, or chemical fusogens such as calcium ions, surface-active lipids, e.g. lysolecithin, or polyethylene glycol (PEG), or by electrofusion. Preferentially, the myeloma cells are fused with the same amount of spleen cells from immunized mammals in a solution containing about 30% to about 60% of polyethylene glycol of a molecular weight between 1000 and 4000.

After the fusion, the cells are resuspended and cultivated in a selective medium chosen depending on the genetic selection marker, for example HAT medium. In this medium, only hybridoma cells will survive, because they combine the ability to grow and replicate in vitro inherited from the parent myeloma cells and the missing HGPRT or TK genes essential for the survival in HAT medium inherited from the antibody-producing spleen cells of the immunized mammals.

Suitable culture media for the expansion of hybridoma cells are the standard culture media, such as Dulbecco's Modified Eagle Medium (DMEM), minimum essential medium, RPMI 1640 and the like, optionally replenished by a mammalian serum, e.g. 10 to 15% fetal calf serum. Preferentially, feeder cells, e.g. normal mouse peritoneal cells, spleen cells, bone marrow macrophages or the like, are added at the beginning of cell growth immediately after the fusion step to nourish the hybridoma cells and support their growth, especially where cell densities are low, by providing growth factors and the like. If phagocytic cells such as macrophages or monocytes are used, they can perform a helpful service in cleaning up the debris of dead myeloma cells always found after aminopterin treatment. The culture media are supplemented with selective medium in order to prevent myeloma cells from overgrowing the hybridoma cells.

The hybridoma cell culture supernatants are screened for the desired monoclonal antibodies with an immunoassay, preferentially a radio- or enzyme immunoassay such as a dot assay. For example, antigen is dotted on nitrocellulose discs, incubated with culture fluids of growing hybridomas, alkaline phosphatase labelled anti-mouse IgG and a substrate solution. The presence of the desired antibodies is indicated by development of a coloured dot.

Positive hybridoma cells are cloned, e.g. by limiting dilution or in soft agar, preferentially twice or more. Optionally, hybridoma cells are passaged through animals, e.g. mice, by intraperitoneal injection and harvesting of ascites, which stabilizes hybridomas and improves growth characteristics. The cloned cell lines may be frozen in a conventional manner.

The invention also relates to processes for the preparation of transfectoma cell lines secreting chimeric anti-p24 monoclonal antibodies as described hereinbefore, characterized in that a suitable host cell is transformed with one or two vectors or unlinked DNA as described hereinbefore, the transformed cells obtained are cloned, and cell clones secreting the desired chimeric monoclonal antibodies are selected.

Vectors or unliked DNAs are introduced into the host cells by conventional techniques, such as calcium phosphate precipitation, microinjection into the cell nucleus, protoplast fusion, electroporation, i.e. introduction of DNA by a short electrical pulse which transiently increases the permeability of the cell membrane, or the like. Transfection may be carried out in the presence of helper compounds, e.g. diethylaminoethyldextran, dimethyl sulfoxide, glycerol, polyethylene glycol or the like, or as co-precipitates of vector DNA and calcium phosphate.

After the transfection procedure, transfected cells are identified and selected with the help of a selection procedure matching the selection marker of the DNA used for transfection. Selection markers include genes which confer resistance to heavy metals such as copper or to antibiotics, e.g. G-418 (geneticin, a neomycin-derivative) or hygromycin, or genes which complement a genetic lesion of the host cell such as the absence of thymidine kinase, hypoxanthine phosphoribosyl transferase, dihydrofolate reductase, or the like. For example, if the DNA used for transfection comprises a marker for geneticin resistance, transformed cells are identified and separated from untransformed cells by culture in the presence of the antibiotic geneticin.

Cultivation, selection and cloning are performed as described above for hybridoma cell lines

The monoclonal antibodies and their derivatives according to the invention are useful for a number of therapeutic and diagnostic purposes.

In particular, the monoclonal antibodies and their derivatives can be used for the prevention of the progression of AIDS. Hence, the decrease in anti-p24 antibodies frequently observed six months to one year before the transition from the asymptomatic to the symptomatic stage of the disease is counteracted. The protective effect of anti-p24 antibodies is connected with the expression of p24 on the surface of infected cells, thus providing a target for cell-mediated immune cytotoxicity. Also, the monoclonal antibodies and derivatives thereof of the invention are useful for the treatment of HIV infection, because they selectively kill HIV infected cells, for example by ADCC, inhibit the spread of infection from HIV-infected to non-infected cells, for example by preventing the production of HIV particles by HIV-infected cells, and/or reduce the amount of infectious HIV produced by macrophages and chronically infected lymphoid cells. Due to their reduced immunogenicity, the chimeric monoclonal antibodies and derivatives thereof according to the invention are especially useful for therapeutic applications and prophylaxis.

The invention also concerns pharmaceutical compositions for passive or active immunization, i.e. the prevention of the progression or the treatment of AIDS, comprising a therapeutically effective amount of a monoclonal antibody and/or of a derivative thereof according to the invention and a pharmaceutically accetable carrier.

Preferred are pharmaceutical compositions for parenteral application and inhalation, e.g. nasal application. Compositions for intramuscular, subcutaneous or intravenous application or for inhalation are e.g. isotonic aqueous solutions or suspensions, optionally prepared shortly before use from lyophilized or concentrated preparations. Suspensions in oil contain as oily component the vegetable, synthetic or semi-synthetic oils customary for injection purposes. The pharmaceutical compositions may be sterilized and contain adjuncts, e.g. for conserving, stabilizing, wetting, emulsifying or solubilizing the ingredients, salts for the regulation of the osmotic pressure, buffer and/or compounds regulating the viscosity, e.g. sodium carboxycellulose, carboxymethylcellulose, sodium carboxymethylcellulose, dextran, polyvinylpyrrolidine or gelatine.

The pharmaceutical compositions of the invention contain from approximately 0.01 % to approximately 50% of active ingredients. They may be in dosage unit form, such as ready-to-use ampoules or vials, or also in lyophylized solid form.

In general, the therapeutically effective dose for humans is between approximately 100 and 500 µg of monoclonal antibodies of the invention and/or derivatives thereof per kg body weight depending on the status of the patient and the mode of application. The specific mode of administration and the appropriate dosage will be selected by the attending physician taking into account the particulars of the patient, the state of the disease, the type of autoimmune disease or immunological disorder treated, and the like.

The pharmaceutical compositions of the invention are prepared by methods known in the art, e.g. by conventional mixing, dissolving, confectioning or lyophilizing processes. Pharmaceutical compositions for injection are processed, filled into ampoules or vials, and sealed under aseptic conditions according to methods known in the art.

The pharmaceutical compositions may also be for enteral, such as rectal or oral, administration. Pharmaceutical compositions for oral application can be obtained by combining the active ingredient with solid carriers, optionally granulating a resulting mixture and, if desired or necessary after the addition of suitable adjuncts, processing the mixture or granulate into tablets or dragée cores. They can also be incorporated into plastics carriers which release the active ingredients, or allow them to diffuse, in a controlled manner.

In addition, the monoclonal antibodies and their derivatives can be employed for the diagnosis of HIV infection by the qualitative and quantitative determination of HIV p24 in an immunoassay.

In general, the monoclonal antibodies or derivatives thereof according to the invention can be used in any of the known immunoassays which rely on the binding interaction between epitopes of p24 and the idiotopes of the antibodies directed against p24. Examples of such assays are radio-, enzyme, fluorescence, chemiluminescence, immunoprecipitation, latex agglutination, and hemagglutination immunoassays.

The monoclonal antibodies according to the invention can be used as such or in the form of radioactively labelled derivatives in a radioimmunoassay (RIA). Any of the known modifications of a RIA can be used, for example soluble phase (homogeneous) RIA, solid phase (heterogeneous) RIA, single RIA or double (sandwich) RIA with direct or indirect (competitive) determination of HIV p24. An example of such a radioimmunoassay is a sandwich RIA in which a suitable carrier, for example the plastic surface of a microtiter plate or of a test tube, e.g. of polystyrene, polypropylene or polyvinylchloride, glass or plastic beads, filter paper, dextran etc. cellulose acetate or nitrocellulose sheets, magnetic particles or the like, is coated with a monoclonal antibody of the invention by simple adsorption or optionally after activation of the carrier, for example with glutaraldehyde or cyanogen bromide. Then test solutions containing p24 and finally polyclonal antibodies which react with a different epitope of p24 and which are radioactively labelled, e.g. with $^{125}$I, are added. The amount of antibodies directed against p24 in the test solution is directly proportional to the amount of bound polyclonal antibodies and is determined by measuring the radioactivity of the solid phase.

The monoclonal antibodies according to the invention can be used as such or in the form of enzyme-conjugated derivatives in an enzyme immunoassay. As described above for radioimmunoassays, any of the known modifications of an enzyme immunoassay can be used.

The tests are carried out in an analogous manner to the radioimmunoassays decribed above using an enzyme label instead of a radioactive label. The amount of immune complex formed which corresponds to the amount of p24 present in the test solutions is determined by adding an enzyme substrate solution. The enzyme substrate reaction results, for example, in a color change which can be observed by eye or with optical measuring devices.

The monoclonal antibodies according to the invention can be used as such or in the form of derivatives conjugated with chemiluminescent markers in a chemiluminescence immunoassay. As described above for radioimmunoassays, any of the known modifications of. a chemiluminescence immunoassay can be used.

The tests are carried out in an analogous manner to the radioimmunoassays described above using a chemiluminescent label instead of a radioactive label. The amount of immune complex formed which corresponds to the amount of p24 present in the test solutions is determined by adding a compound triggering lu-

EP 0 519 866 A1

minescence, e.g. $H_2O_2$ and NaOH, and measuring the emission of light with optical measuring devices.

The use according to the invention of monoclonal antibodies and derivatives thereof as described hereinbefore for the determination of p24 also includes other immunoassays known per se, for example immunofluorescence assays, latex agglutination, hemagglutination, evanescent light assays using an optical fibre coated with an antiidiotypic MAb and other direct-acting immunosensors which convert the binding event into an electrical or optical signal, or the like.

The invention also concerns test kits for the qualitative and quantitative determination of HIV p24 comprising monoclonal antibodies of the invention and/or derivatives thereof and, optionally, other polyclonal or monoclonal antibodies and/or adjuncts.

Test kits according to the invention for a radioimmunoassay contain, for example, a suitable carrier, optionally freeze-dried solutions of one or more polyclonal and/or monoclonal antibodies, solutions of a radioactively labelled antibody, standard solutions of p24, buffer solutions, and, optionally, polypeptides or detergents for preventing non-specific adsorption and aggregate formation, pipettes, reaction vessels, calibration curves, instruction manuals and the like. One of the antibodies of the test kit is a monoclonal antibody of the invention.

Test kits according to the invention for an enzyme immunoassay contain, for example, a suitable carrier, optionally freeze-dried solutions of one or more polyclonal and/or monoclonal antibodies, optionally freeze-dried or concentrated solutions of an enzyme- or biotin-conjugated antibody, solutions of an enzyme-avidin conjugate if biotin-labelled antibody is used, enzyme substrate in solid or dissolved form, standard solutions of p24, buffer solutions, and, optionally, polypeptides or detergents for preventing non-specific adsorption and aggregate formation, pipettes, reaction vessels, calibration curves, instruction manuals and the like. One of the antibodies of the test kit is a monoclonal antibody of the invention.

The following examples illustrate the invention but do not limit it to any extent.

### Abbreviations

| | |
|---|---|
| BSA | bovine serum albumin |
| DAPI | 4',6'-diamino-2-phenylindole |
| DMEM | Dulbecco's modified Eagle medium |
| DTT | dithiothreitol |
| EBSS | Earle's buffered salt solution |
| FCS | foetal calf serum |
| HAT | hypoxanthine, aminopterin and thymidine |
| HEPES | N-2-hydroxyethyl-piperazine-N'-2-ethanesulfonic acid |
| HT | hypoxanthine and thymidine |
| IFN | interferon |
| I1 | interleukin |
| MAb | monoclonal antibody |
| NC | nitrocellulose |
| PEG | polyethylene glycol |
| PBS | phosphate buffered saline |
| TBST | 10 mM Tris/HCl, pH 8.0, 150 mM NaCl, 0.05 % Tween™ 20 |
| U | unit |

### Examples

### Example 1 Preparation of hybridoma cell lines

### 1.1 Preparation of antigen for immunization:

Antigen mixture I consists of 20 µg recombinant HIV core antigen p25 (lot Nr. 69CO2J, Chiron), 10 µg adjuvant peptide (N-acetylmuramyl-L-alanyl-D-isoglutamine, Sigma), 5000 U recombinant Il-2 (Kyowa Hakko, Tokyo, Japan), 1000 U recombinant rat-IFNγ (H. Schellekens, Primate Center TNO, Rijswijk, The Netherlands) and 200 µl Complete Freund's adjuvant (CalBiochem) in a total volume of 400 µl. Antigen mixture II consists of 10 µg HIV antigen p25, 10 µg adjuvant peptide, 5000 U Il-2, 1000 U rat-IFNγ and 150 µl Incomplete Freund's Adjuvant (CalBiochem) in a total volume of 300 µl. Antigen mixture III consists of 50 µg HIV antigen p25, 10 µg adjuvant peptide, 5000 U Il-2 and 1000 U rat-IFNγ in a total volume of 150 µl (no Freund's Adjuvant).

14

### 1.2 Immunization schedule:

Four week old Balb/c mice are immunized with antigen mixture I by subcutaneous (s.c.) injection in the neck region followed by four booster injections s.c.in different back and leg regions using antigen mixture II on days 7, 14, 21 and 28. The final injection of antigen mixture III is given intraveneously on day 43, three days before fusion.

### 1.3 Cell fusion:

The spleen of an immunized mouse sacrificed on day 46 is homogenized in Earle's buffered salt solution (EBSS, without $Ca^{2+}$ and $Mg^{2+}$; Gibco), and the suspension is allowed to settle for 15 min to remove tissue debris. The cells in the supernatant are pelleted by centrifugation (300 x g, 10 min), and washed once with EBSS. $10^8$ spleen cells are mixed with $10^8$ Sp2/0-Ag14 mouse myeloma cells and pelleted by centrifugation (300 x g, 10 min). Sp2/0-Ag 14 (ATCC CRL 1581 ) is a well-characterized mouse cell-line of lymphoid origin. It is an Ig non-secreting variant of a cell-line obtained from the fusion of a mouse spleen cell with the myeloma X63-Ag8, a subline of the myeloma MOPC-21 (Köhler and Milstein, Eur. J. Immunol. $\underline{6}$, 511, 1976; Shulman et al., Nature $\underline{276}$, 270, 1978). 1 ml of freshly thawn and prewarmed (37°C) polyethylene glycol solution (PEG 1450, Kodak, 50% w/v in PBS) is slowly added to the cell pellet while gently shaking at 37°C. After subsequent 90 seconds incubation at 37°C without shaking, 1 ml EBSS is added, and the mixture incubated for 3 min at room temperature. Further addition of 10 ml EBSS and incubation at room temperature for 5 min is followed by centrifugation (300 x g, 10 min) and resuspension of the cell pellet in 100 ml of selection medium consisting of 0,1 mM hypoxanthine, 0,4 μM aminopterin and 16 μM thymidine (HAT, Boehringer Mannheim) in HB 101 (AMS Biotechnology), 5% CLEX™ (Dextran Products Ltd., Canada), 4 mM glutamine (Seromed), 100 U/ml penicillin and 100 μg/ml streptomycin (Seromed), 1 mM sodium pyruvate (Seromed), and 10 mM HEPES (Seromed). The cell suspension is seeded at 100 μVwell in ten 96 well plates preplated with 100 μl/well of a further layer of murine (Balb/c) peritoneal resident cells (4000 cells per well), and incubated at 37°C, 8 % $CO_2$ for 12 to 20 days. One week after seeding 50 μl of fresh HAT selection medium are added. Starting on day 12 the plates are examined microscopically for detection of growing colonies. The supernatants of growing colonies (hybridomas) are tested for presence of anti-p25 antibodies as described in example 1.5.

### 1.4 Processing and subcloning procedure of positive hybridomas:

Once positive hybridomas are selected cells are transferred into HT medium (same composition as selection medium but without aminopterine) in 24 well plates. One week later survivors are transferred into hybridoma medium (same composition as selection medium but without HAT and with 2 % instead of 5 % CLEX™), and cultured for further antibody production. Positive hybridomas are maintained in culture and controlled for continuous production of anti-p25 antibodies. After expansion aliquots of cells are frozen at -80°C in 95 % FCS (Seromed), 5% dimethyl sulfoxide-d6 (DMSO, Dr. Glaser AG, Basel), and stored in liquid nitrogen. Subcloning of positive hybridomas is performed by dilution of the cell suspension to 3 cells/ml followed by distribution at 100 μl per well on ten microtiter plates in presence of feeder cells (murine peritoneal cells). The supernatants of growing clones are rescreened for antibody production as described in example 1.5. Positive clones are maintained, expanded, and the subtypes of the antibodies in the culture fluids are characterized as described in example 1.6.

### 1.5 Antibody detection assay:

Specific murine antibodies produced by growing hybridomas are discovered by testing the supernatants in a "dot-ELISA":

0.5 μl PBS containing 50 ng p25 antigen are dotted on small nitrocellulose discs (NC-discs with diameter of 0,5 cm, prepared from sheets of NC Type HA 0,45 μm, Millipore™) fitting into the wells of microtiter plates, using an automatic dispenser (Microlab M™, Hamilton). Antigen containing discs are fixed with 0,25 % glutaraldehyde in PBS, washed three timeswith PBS, distributed in microtiter plates, and air dried. The dried discs can be stored for several weeks at 4°C before use. Anti-p25 antibodies are detected in culture fluids of growing hybridomas by adding 100 μl of the supernatants to the NC-discs that previously have been blocked with 3% BSA (Sigma) and 10% horse serum (Seromed) in 10 mM Tris/HCl, pH 8.0, 150 mM NaCl, 0,05 % Tween™ 20 (TBST). After incubation of the NC-discs with the hybridoma supernatants (2 h, 37°C or overnight at 4°C) the NC-discs are washed 5 times with TBST, followed by incubation for 2 h at 37°C with alkaline phosphatase labelled anti-mouse IgG (Promega, Lot B 207 A) diluted 1: 7000 in TBST. After 5 washes with TBST 100 μl substrate

solution are added; the substrate solution is prepared freshly by mixing 10 ml substrate buffer (100 mM Tris/HCl, pH 9.6, 150 mM NaCl, 5 mM $MgCl_2$) with 66 $\mu$l NBT-solution (60 mM Nitrotetrazolium Blue chloride, Fluka, in 70 % N,N-dimethylformamide, Fluka) and with 33 $\mu$l BCIP-solution (135 mM 5-bromo-4-chloro-3-indolyl phosphate p-toluidine salt, Fluka, in N,N-dimethylformamide). The presence of anti-p25 antibodies is indicated by development of a dark blue-violet coloured dot on the NC-disc.

### 1.6 Subtype determination of anti-p25 antibodies:

The subclass of anti-p25 monoclonal antibodies is determined as follows: 800 $\mu$l of MAb-containing cell culture supernatant are distributed in 8 wells of a microtiter plate containing p25 dotted NC-discs blocked with 3% BSA, 10% horse serum in TBST. After incubation for 2 h at 37°C or overnight at 4°C the NC-discs are washed 5 times with TBST followed by 2 h incubation with 50 $\mu$l/disc of subtype-specific rabbit-anti-mouse Ig Abs (BIO-RAD murine monoclonal antibody subtyping kit) at 37°C and five washes with TBST. 100 $\mu$l/well of alkaline phosphatase-labelled goat-anti-rabbit IgG (H+L; BIO-RAD No. 172-1016, 1:2000 in TBST) are added, and the mixture incubated for 2 h at 37°C followed by 5 washes with TBST. The colour development occurs in the presence of 100 $\mu$l per well substrate solution (NBT/BCIP; see example 1.5) at room temperature.

### 1.7 Selection of anti-p25 MAbs:

Following the procedure of example 1.4 a panel of 142 anti-p25 MAb producing subclones of different subtypes (IgM, IgA, IgG1, IgG2a, IgG2b, IgG3, all containing $\kappa$-light chains) is obtained. No crossreactivity to HIV *env* protein gp41 is found for any of these antibodies when tested in a dot-ELISA of example 1.5 wherein p25 is replaced by gp41 (Chiron). Titers of the different antibodies are evaluated in a limiting dilution titration, and the lowest concentrations necessary for detection of p25-dots are calculated. The monoclonal antibodies named 25-57-1 and 26-69-5 (both IgG1, $\kappa$-light chain) are found to be strong binding antibodies. They can be diluted down to 0.1 - 1 ng/ml (as determined in eight different experiments), while for most other anti-p25 antibodies the range is between 5 and 15 ng/ml. Therefore MAb 25-57-1 and MAb 26-69-5 are selected for further characterization and large scale production.

### 1.8 Large scale production of MAbs:

Monoclonal antibodies are produced in large scale amounts using the Dynacell™ Culture System (Millipore), a membrane based perfusion system for production of monoclonal antibodies by hybridoma cells. 3-$5x10^7$ hybridoma cells are placed into the cell module, a chamber containing alternate layers of microporous membranes. The cells are trapped between adjacent membranes, and nourished with hybridoma medium (HB 101; AMS Biotechnology), 4 mM glutamine (Seromed), 100 U/ml penicillin and 100 $\mu$g/ml streptomycin (Seromed), 1 mM sodium pyruvate (Seromed), and 10 mM HEPES (Seromed), optionally supplemented with 5% CLEX™ (Dexaan Products Ltd., Canada), which circulates through the module from a reservoir (1 1) via a peristaltic pump. MAbs secreted by the cells are accumulated in this reservoir. Each 4-7 days MAb containing medium is harvested, and fresh medium is added. The cells can be kept in this system producing MAbs for up to six months. Antibody concentration in the supernatant is determinded as described in example 1.10.

During a time period of 83 days, a total amount of 280 mg MAb 26-69-5 can be produced. IgG concentrations in the supernatants harvested every few days are in the range of 0.7-52 $\mu$g/ml. Periods of low productivity (< 4 $\mu$g/ml) from day 6 to day 31 and from day 54 to day 63 are followed by periods of high productivity with MAb concentrations of 5-50 $\mu$g/ml IgG.

### 1.9 Enrichment of MAbs in Dynacell™ Culture System supernatants:

Monoclonal antibodies in hybridoma supernatants are concentrated by tangential flow ultrafiltration using the Minitan™ Acrylic System (Millipore). The supernatants are filtrated through a stack of polysulfone membranes (PTHK Minitan™ plates, Millipore) with a molecular weight exclusion limit of 100 kDalton in order to retain the IgG fraction. The filtrate is discarded while the retentate contains the concentrated antibody sample. 8-10 subsequent Dynacell™ Culture System supernatants are pooled (8-10 1) and concentrated to 200-250 ml retentate. Enrichment of IgG (100 - 400 $\mu$g/ml) is determined by a limiting dilution dot ELISA as described in example 1.10.

**1.10** Limiting dilution dot ELISA for evaluation of MAb concentration:

The concentration of monoclonal antibodies in hybridoma supernatants (or in retentate after enrichment with the Minitan™ Acrylic System) is evaluated as follows: The sample and a standard solution of known murine IgG concentration (10 µg/ml) are diluted in two-fold or in three-fold serial dilutions in PBS ($2^{-1}$ to $2^{-12}$ and $3^{-1}$ to $3^{-12}$). The serial dilution samples are transferred to a NC-sheet as 1 µl dots and air dried. After incubation with blocking buffer (3 % BSA, 10 % horse serum in TBST) for 1 h at room temperature, alkaline phosphatase-labelled anti-murine Ig antibody (Promega, diluted 1: 7000 in blocking buffer) is incubated with the NC-sheet for 1 h at room temperature followed by 5 washes with TBST. Staining of the dots is performed with NBT/BCIP substrate solution (example 1.5) for 10-15 min followed by two washes with water. The IgG concentration of a sample is evaluated by comparing the highest dilution of the sample still detectable as stained dot with that of the standard solution. The IgG concentration of the samples is calculated as a mean value from the two-fold and the three-fold serial dilutions.

**Example 2** Assays for biologic activity

**2.1** Cellular antibody binding assay:

Monoclonal antibodies are tested for binding to natural antigen p24 using HIV-infected human macrophages as a target. Macrophages are prepared as described in J.K. lazdins et al., AIDS Research and Human Retroviruses 6, 1203 (1990) using a combination of lymphocytapheresis and countercurrent elutriation to obtain 95 % pure monocytes that are allowed to differentiate in vitro by culturing them in bacteriological grade Petri dishes for 10-15 days in complete macrophage medium consisting of DMEM high glucose 4,5 g/l (Gibco), 10 % human type AB non heat inactivated serum (Sigma), 50 U/ml penicillin and 50 µg/ml streptomycin (Amined, Basel, Switzerland), 2 mM L-glutamine, and 1 mM sodium pyruvate (Gibco). The macrophages are detached and replated in 96 well plates at $6 \times 10^4$ cells per well. The monolayers are infected with monocytotropic HIV-$1_{ADA}$ (obtained through the AIDS Research and Reference Reagent Program, Division of AIDS, NIAID, NIH, from Dr. H. Gendelman; the virus is passaged in in vitro differentiated blood monocytes as described in J.K. Lazdins et al., loc. cit.) and cultured at 37°C, 5 % $CO_2$, until the formation of giant cells is visible.

After removal of supernatants the cells are fixed with 2 % formaldehyde in PBS for 30 min at room temperature, and washed once with PBS. For permeabilization the cells are incubated with Nonidet™ P-40 (Sigma, 0,5 % in PBS) for 5 min followed by two washes with PBS containing 10 mM glycine. For surface staining, no permeabilization is needed. Blockinng buffer (10 % human serum, 0.2 % gelatine, 0.05 % Tween™ 20, 1 % BSA) is then added for 30 min at room temperature to saturate unspecific binding sites. After removal of blocking buffer, the cells are incubated with hybridoma supernatants (anti-p25) or appropriate control antibodies for 1 h at 37°C followed by three washes with NKH (15 mM NaCl, 3 mM KCl, 2 mM HEPES, pH 7.3). Alkaline phosphatase-labelled anti-mouse-IgG antibody (F(ab')$_2$ goat anti-mouse IgG, Jackson Immunoresearch Lab.) diluted 1:300 in blocking buffer is added, and the mixture incubated for 30 min at 37°C. After three washes with NKH and one wash with substrate buffer (0.1 M Tris/HCl, 5 mM MgCl$_2$, 100 mM NaCl, pH 8.8) substrate (1 mg/ml Fast Red, BIO-RAD, 0,4 mg/ml naphthol phosphate, BIO-RAD, 1 mM levamisole, Sigma) is added and allowed to develop in the dark for 15-20 min. The reaction is stopped by washing with PBS, and stained samples are examined in a light microscope or, if necessary, stored in PBS with 0.1 % NaN$_3$ at 4°C for several days.

**2.2** Cellular assays:

$5 \times 10^4$ human macrophages per well are plated into 96 well plates, infected with HIV-$1_{ADA}$ (J.K. Lazdins et al., loc. cit.) and incubated at 37°C, 5% $CO_2$. Macrophage medium (see example 2.1) is changed every 3 days. When the infection is well established as seen by formation of giant cells, supernatants are removed and replaced by either fresh medium (control) or medium containing anti-p25 MAb to be tested. During further incubation at 37°C, 5% $CO_2$, samples of supernatants (10 µl) are taken in triplicate for determination of reverse transcriptase production and frozen at -20°C until the reverse transcriptase assay of example 2.3 is performed. Samples of one experiment are assayed all at once to reduce inter-assay variability.

H9 cells that are persistently infected with HIV-$1_{III-B}$ (H9/HTLV-III$_B$ NIH 1983, obtained from the AIDS Research and Reference Reagent Program, NIH, Dr. Robert Gallo) are grown in H9 medium consisting of RPMI1640 (Seromed), 10 % FCS (Seromed), 100 U/ml penicillin and 100 µg/ml streptomycin (Seromed), 2 mM L-glutamine (Seromed) and 10 mM HEPES (Seromed), and passaged every 3 to 4 days to $1 \times 10^5$ cells/ml. For the experiments, cells are washed and resuspended in fresh medium and dispensed in U-bottom 96 well plates at $1.5 \times 10^4 - 5 \times 10^4$ cells/well. Anti-p25 MAb to be tested or control murine IgG (Cappell) are mixed with the cells in triplicates followed by incubation at 37°C, 5 % CO$_2$. 10 μl samples are taken for the reverse transcriptase assay each following day and frozen at -20°C until the reverse transcriptase assay is performed.

MT-2 cells transformed with HTLV-I and continuous producer of HTLV-I, line cloned for maximal cytopathic effects with LAV-I (obtained from the AIDS Research and Reference Reagent Program, NIH, Dr. Douglas Richman), are grown in H9 medium and passaged every 3 - 4 days. For coculture experiments, H9 cells are washed with medium and resuspended in fresh medium or medium containing anti-p25 MAb to be tested. After 4-18 h incubation at 37°C, 5% CO$_2$, the cells are washed again, and 300 of the pretreated H9 cells are mixed with 3 x 10$^4$ fresh MT-2 cells in absence or presence of anti-p25 MAb to be tested in a microtiter plate in triplicates, then incubated at 37°C, 5% CO$_2$. Samples for the reverse transcriptase assay are taken each day and frozen at -20°C until the reverse transcriptase assay is performed.

2.3 Reverse transcriptase assay:

HIV reverse transcriptase activity is evaluated as described by F. Di Marzo Veronese et al., Science 231, 1289 (1986). The reverse transcriptase reaction mixture contains 50 mM Tris (ultra pure, BRL), pH 7.8, 75 mM KCl (Baker), 2 mM DTT (Cleland's reagent, CalBiochem), 5 mM MgCl$_2$ (Mallinckrodt), 50 μg/ml Poly(A) (polyadenylic acid, Pharmacia), 1,6 μg/ml pd(T)$_{12-18}$ (Oligodeoxythymidylic acid, Pharmacia) and 0,05 % NP-40, is freshly prepared, filtered through a 0,45 micron Acrodisc™ filter (Gelman Sciences), and stored in 5 ml aliquots at -20°C. For assaying reverse transcriptase, the mixture is thawn and mixed with 10 mCi/ml $^{32}$P-TTP (thymidine 5′-[α-$^{32}$P]triphosphate triethylammonium salt, Amersham) at a ratio of 1000 : 1 to give a final activity of 10 μCi/ml. 10 μl samples in triplicates are taken from culture supernatants and transferred to U-bottom 96-well plates. Samples can be used immediately or stored at -20°C. When kinetics of reverse transcriptase production is studied, samples are frown immediately after harvesting and assayed at once at the end of the experiment to avoid inter-assay variability. 50 μl/well of complete reverse transcriptase cocktail is added to the samples, mixed, and incubated for 1.5 to 3 h at 37°C. 5 μl of this reaction mixture is then dotted on DEAE-paper (DE81, Whatman) and air dried. Four washes (5 min each) with 2 x SSC (300 mM NaCl, 25 mM sodium citrate) are followed by two washes (1 min) with 95 % ethanol and air drying. The dotted spots are visualized by autoradiography and/or counted in a scintillation counter (Beckman, LS 1801).

2.4 Staining of nuclei of macrophages with DAPI:

Monolayers of infected or uninfected macrophages in flat bottom 96 well plates are fixed with 2 % formaldehyde-PBS for 30 min at room temperature. After washing 0.5 μg/ml DAPI (4′,6′-diamino-2-phenylindole, Serva Fein Biochemica, Heidelberg) in McIlvaines buffer pH 4.5 (53 mM citric acid, 94 mM Na$_2$HPO$_4$, 10 mM MgSO$_4$) is added to the cells, and the mixture incubated for 2 h (room temperature) in the dark. The fluorescence of dye bound to the A-T rich regions of DNA in the nuclei is measured in a fluorimeter (Titertek Fluoroskan™ II) at 355 nm excitation and 460 nm emission. The results are expressed as arbitrary fluorescence units per well.

2.5 ELISA for determination of cell associated viral antigen in macrophages:

The procedure for the quantitative evaluation of cell associated viral antigen in infected macrophages is essentially the same as described in example 2.1 (binding to natural antigen) with two exceptions: The alkaline phosphatase conjugated second antibody is used at a 1 : 2500 dilution, and p-nitrophenyl phosphate (Sigma, one substrate tablet per 5 ml) in diethanolamine buffer (10 % diethanolamine, 0.5 % MgCl$_2$, 0.02 % NaN$_3$, pH 9,6) is used as substrate. Colour development is determined at 405 nm with an ELISA reader (Tecan, EIA-autoreader KUCO-21™).

For macrophages which have been pretreated with MAb 26-69-5 (see experiment shown in Table 1), the assay has to be modified. Reagents of the HIV-1 p24 Core Profil ELISA Kit (Dupont) are used for detection of the cell associated p24: After incubation of the cells with the biotinylated polyclonal anti-p24 antibody, the binding is probed with the streptavidin horseradish peroxidase conjugate; the complex is detected with o-phenylenediamine-HCl as substrate. The concentrations of the reagents and the incubation times are used as recommended by the manufacturers of the Dupont kit. The colour development is read at 490 nm with the EIA-autoreader KUCO-21™ (Tecan).

2.6 ADCC assay:

Effector cells (human peripheral blood lymphocytes, PBL) are isolated from human blood as follows: 10 ml heparinized blood are mixed with 10 ml PBS (without $Ca^{++}$, $Mg^{++}$) and underlayered with 15 ml Lymphoprep™ (Nycomed) in 50 ml centrifugation tubes. Following centrifugation (400 x g, 30 min) the peripheral blood lymphocytes forming the interphase band are harvested, washed twice with PBS and resuspended in RPMI medium with 10 % FCS. Viable cells are counted, and the concentration adjusted to $2 \times 10^7$ cells/ml. Target cells are prepared by incubating $1 \times 10^6$ persistently infected H9 cells with 200 μCi (200 μl 1 mCi/ml) $^{51}$chromium ($Na_2CrO_4$, Amersham) for 45 min at 37°C. After three washes with medium the cells are further incubated for 15 min at 37°C followed by an additional washing step in order to reduce unspecific chromium release. Viable labelled cells are counted, and the concentration adjusted to $5 \times 10^4$ cells per ml. After dispensing 50 μl of target cells (2500 cells per well) in a U-bottom 96 well plate, the cells are treated in triplicates with 50 μl/well anti-p25 MAb 26-69-5 at final concentrations of 12, 25 or 50 μg/ml or with murine IgG (50 μg/ml) or medium as controls for 15 min at 4°C. 100 μl effector cell suspension (effector to target ratios, 30 : 1, 60 : 1 or 100 : 1) is added to a final volume of 200 μl and, after incubation at 37°C for 5 hours, the plate is centrifuged at 200 x g for 5 min. The release of $^{51}$chromium is measured by counting 100 μl of the cell free supernatants in a Gamma-counter (Compugamma™ 1282, LKB).

Spontaneous release due to leakage of chromium from the labelled target cells is evaluated after incubation with medium in absence of antibody and effector cells.

Unspecific release due to unspecific killing of target cells by the effector cells is evaluated after incubation with the effector cells in absence of antibody and calculated as follows:

$$\% \text{ unspecific release} = \frac{cpm_{eff} - cpm_{spon}}{cpm_{tot} - cpm_{spon}} \times 100$$

$cpm_{eff}$   = (target cells + effector cells, no Ab)
$cpm_{spon}$ = spontaneous release
$cpm_{tot}$   = total release (Triton™ X-100 lysed target cells)

Total release (100 %) is determined by lysing the labelled target cells with 1 % Triton™ X-100 (Fluka) in a final volume of 200 μl and counting 100 μl of the lysate.

Specific release due to antibody mediated killing of target cells by the effector cells (ADCC) is calculated as follows:

$$\% \text{ specific release} = \frac{cpm_{sam} - cpm_{eff}}{cpm_{tot} - cpm_{eff}} \times 100$$

$cpm_{sam}$   = release in sample (targets + Ab + effectors)

Example 3 Biological activity of MAb 25-57-1 and MAb 26-69-5

3.1 Binding to natural antigen:

Following the procedure of example 2.1, human macrophages are infected with HIV-1$_{ADA}$ and used as targets for immuno-staining with MAb 25-57-1 and MAb 26-69-5. Fixed and NP-40™ permeabilized cells stain strongly with both MAbs indicating large amounts of cytoplasmatic p24 in the infected macrophages, Also fixed non-permeabilized cells stain with MAb 25-57-1 and MAb 26-69-5 indicating that p24 is present on the surface of these cells.

3.2 Effect of MAb 26-69-5 on HIV-infection of human macrophages:

The effect of MAb 26-69-5 on reverse transcriptase production by productively infected human macrophages (examples 2.2. and 2.3) used 21 days post infection is very clear-cut. Reverse transcriptase activities are measured in the supernatants of the infected macrophages on day 1 and day 2 after beginning of treatment with the MAb. On day one a 95 % reduction of reverse transcriptase activity is found in the supernatant of treated cells when compared to untreated cells (361 versus 7125 cpm). The same range of reduction (96.7 %) is observed on day 2 (674 versus 20444 cpm). The reduction of reverse transcriptase production is probably not due to a toxic effect of the antibody on the macrophages since the number of nuclei per well (example 2.4) remains the same as that of untreated cells. One would expect that dead cells would desintegrate rapidly. Reverse transcriptase reduction when normalized by the amount of nuclei is 94.5 % on day one and 96.4 % on day two. Intracellular virus measured as cell associated viral antigen (CAVA, example 2.5) is only weakly reduced after treatment with MAb 26-69-5 (1.804 versus 2.202 arbitrary fluorescence units).

In a second experiment macrophages on day 14 post infection with HIV-1$_{ADA}$ are exposed to anti-p25 MAb 26-69-5 or control murine IgG. IFNα (Roferon, Hoffmann-La Roche AG) is used as a positive control for inhibition of reverse transcriptase production. As compared to untreated or murine IgG-treated macrophages production of reverse transcriptase is strongly reduced in cells treated with MAb 26-69-5. On day 1 and day 2 the observed reduction is comparable to that seen for IFNα, a known inhibitor of HIV production by macrophages. From day 3 on production of reverse transcriptase starts to rise again in MAb 26-69-5 treated cells, and the increase parallels the increase of reverse transcriptase in the controls (Table 1).

Table 1: Kinetics of reverse transcriptase production in HIV-infected human macrophages

|  | Day 1 | Day 2 | Day 3 | Day 4 |
|---|---|---|---|---|
| MAb 26-69-5 (25 µg/ml) | 237 cpm | 465 cpm | 805 pm | 1472 cpm |
| IFNα (200 IU/ml) | 334 cpm | 438 cpm | 525 cpm | 654 cpm |
| Murine IgG (control) (25 µg/ml) | 678 cpm | 1590 cpm | 1906 cpm | 2516 cpm |
| Medium (control) | 582 cpm | 1737 cpm | 2389 cpm | 2998 cpm |

3.3Influence of MAb 26-69-5 on reverse transcriptase production in persistently infected H9 cells:

Persistently infected H9 cells (example 2.2) are treated for 5 h with MAb 26-69-5 or control murine IgG, washed, and incubated for five days in absence or presence of MAb at the same concentrations as used for pretreatment. On day 5, reverse transcriptase activity is measured in the supernatants (example 2.3). Pretreatment alone followed by incubation with straight medium does not have any effect on reverse transcriptase production when compared to that of untreated cells. Presence of antibody 26-69-5 during the 5 day incubation period reduces the yield of reverse transcriptase in the supernatants by 50-75 % as compared to the medium controls or the murine IgG treated controls. The reverse transcriptase acitivites are: medium: 170 cpm; murine IgG: 151 cpm; MAb 26-69-5 at 50 µg/ml: 45 cpm; MAb 26-69-5 at 25 µg/ml: 78 cpm; MAb 26-69-5 at 12.5 µg/ml: 50 cpm; mean of triplicates.

3.4 Influence of MAb 26-69-5 on coculture infectivity of permanently infected H9 cells to non infected MT-2 cells:

The effect of MAb 26-69-5 on the ability of permanently infected H9 cells to infect MT-2 cells is examined by coculturing these cells (example 2.2) in the presence of the antibody. In order to optimize the activity of the MAb, the H9 cells are pretreated with this MAb at the given concentration for 18 hours. The reverse transcriptase activities measured in the supernatants on day 3 of coculture are: MAb 26-69-5 at 50 µg/ml: 40 cpm; MAb 26-69-5 at 25 µg/ml: 61 cpm; MAb 26-69-5 at 10 µg/ml: 168 cpm; MAb 26-69-5 at 5 µg/ml: 206 cpm; medium alone (control):214 cpm. MAb 26-69-5 present during the coculture at concentrations of 25 µg/ml or higher reduces the reverse transcriptase activity found in the supernatants to 25 % of that found in non treated cultures.

In order to determine the specificity of this effect the activity of MAb 25-57-1 and MAb 26-69-5 is compared to an irrelevant murine IgG in a coculture experiment similar to that described above. Reverse transcriptase activity in the supernatants of cocultures is assayed on days 3 and 4 (Table 2). Only presence of MAb 26-69-5 significantly affects reverse transcriptase production while control IgG is not effective. From day 3 to day 4 reverse transcriptase activity in supernatants increases 8 to 10 fold for cultures treated with murine IgG or medium. On the other hand, MAb 26-69-5 treated cells show a 2 fold increase from day 3 to day 4 (Table 2).

Table 2: Influence of MAbs on reverse transcriptase production in coculture of
MT2 cells with antibody pretreated permanently HIV-infected H9 cells

|  | Day 3 | Day 4 |
|---|---|---|
| MAb 26-69-5 (25 µg/ml) | 26 cpm | 46 cpm |
| MAb 25-57-1 (25 µg/ml) | 117 cpm | 977 cpm |
| polyclonal murine IgG (25 µg/ml) | 143 cpm | 1147 cpm |
| medium (control) | 154 cpm | 1508 cpm |

3.5 ADCC assay:

MAb 25-57-1 and MAb 26-69-5 recognize surface p24 antigen and are of subtypes (IgG2b and IgG1, respectively) that are known to mediate ADCC with human effector cells. The unspecific release due to killing of target cells by effector cells in absence of antibody is calculated as 30% (effector to target ratio 100:1), 23% (60:1), and 17% (30:1). Specific release due to antibody-mediated killing of target cells by the effector cells is observed with all effector to target ratios in presence of anti-p25 MAb 25-57-1 and MAb 26-69-5 but not in presence of control murine IgG (Table 3). ADCC mediating activity of the anti-p25 MAb is observed at all antibody concentrations in a dose dependent fashion down to the lowest concentration examined (12 µg/ml).

Table 3: ADCC mediating activity of the MAbs

| Antibody | Effector to target ratio | | |
|---|---|---|---|
|  | 30:1 | 60:1 | 100:1 |
| MAb 25-57-1 |  |  |  |
| 12 µg/ml | 9.0 * | 13.9 | 13.0 |
| 25 µg/ml | 13.0 | 19.7 | 15.8 |
| 50 µg/ml | 15.7 | 17.9 | 20.3 |
| MAb 26-69-5 |  |  |  |
| 12 µg/ml | 7.1 | 7.6 | 9.1 |
| 25 µg/ml | 9.7 | 14.0 | 11.0 |
| 50 µg/ml | 11.7 | 17.4 | 18.3 |
| murine IgG |  |  |  |
| 50 µg/ml | -1.4 | -0.3 | -1.1 |

* specific release (%)

Example 4.Cloning and adaptation of functional H- and L-chain V-region exonsof murine hybridomas 25-57-1 and 26-29-5 for expression in Sp2/0 myeloma cells

General methods used are described in detail in Sambrook et al. (Molecular Cloning: A Laboratory Manual, Second Edition, Cold Spring Harbor Press, 1989).

4.1 Origin of murine hybridomas 25-57-1 and 26-29-5 and preparation of RNA:

The manufacture and properties of the murine hybridomas 25-57-1 and 26-29-5 are described in examples 1 and 3. Total RNA is isolated from ca. $0.5\text{-}1\times10^8$ cells using the procedure described by Le Meur et al. (Cell 23, 561-571, 1981).

4.2 In vitro amplification of functional H- and L-chain Ig cDNA sequences:

Nucleotide sequences corresponding to the coding regions of Ig genes expressed in the hybridomas 25-57-1 and 26-29-5 are obtained by reverse transcription of total hybridoma RNA, and amplification in vitro of the Ig cDNA transcripts using Taq DNA polymerase. The following oligonucleotide primers are used (letters in brackets signify degenerate nucleotides at these positions):

VH1FOR:    5'-TGAGGAGACGGTGACCGTGGTCCCTTGGCCCCAG-3'

VH1BACK: 5'-AGGT(G/C)(C/A)A(G/A)CTGCAG(G/C)AGTC(T/A)GG-3'

VK1FOR:    5'-GTTAGATCTCCAGCTTGGT(G/C)C(G/C)-3'

VK1BACK: 5'-GACATTCAGCTGACCCAGTCTCCA-3'

M/Cκ:        5'-GGGAAGATGGATACAGTTGG-3'

M/Cκ corresponds to the reverse antisense strand of the mouse Ig L-chain Cκ constant region exon (Hieter et al., Cell 22, 197-207, 1980). VH1FOR, VH1BACK, VK1FOR and VK1BACK correspond to the Ig H- and L-chain V-region primers described by Orlando et al. (Proc. Natl. Acad. Sci. USA 86, 3833-3837, 1989), with the exception that VK1FOR used here includes two degenerate base substitutions (underlined above). The latter set of primers include DNA restriction sites which facilitate later cloning of amplified V-regions (see example 4.3). These are: VH1FOR: BstEII (GGTGACC); VH1BACK: PstI (CTGCAG); VK1FOR: BglII (AGATCT); VK-1BACK: PvuII (CAGCTG).

For reverse transcription and amplification of Ig H-chain mRNA, total RNA (10 μg) from hybridomas 25-57-1 and 26-29-5 is treated for 90 min at 37°C with 200 units of MMLV Reverse Transcriptase (1 μl; Gibco-BRL) in a solution containing 10 μl of RT-buffer, 14 μl of $H_2O$, 2.5 μl of spermidine (10mM), 0.5 μl of BSA (10 mg/ml), 10 μl of mixed dNTP (2 mM each; N = A, T, G and C), 10 μl of Triton X-100™ (10%, v/v), 1.5 μl of RNAse Block™ (Stratagene) and 1 μl of VH1FOR primer (50 pmol).

A portion (5 μl) of this solution containing Ig cDNA is subjected to in vitro amplification in 100 μl of solution containing: 62.5 μl $H_2O$, 10 μl of PCR-buffer, 10 μl of a mixture of dNTP (2 mM each, N = A, T, G and C), 10 ml of dimethyl sulphoxide (Merck) and 2 μl of primers VH1FOR and VH1BACK (50 pmol in $H_2O$). The solution is mixed and heated to 93°C for 3 min, cooled to 37°C, 0.4 μl of AmpliTaq™ DNA polymerase (Perkin Elmer Cetus) are added, and the solution is overlaid with 100 μl of paraffin oil in a 1 ml Eppendorf™ tube. The solution is then incubated in a temperature cycler (Intelligent Heating Block, Hybaid) as follows: 71°C for 0.2 min, followed by 93°C for 0.01 min, followed by 37°C for 0.2 min (4 cycles); 71°C for 0.2 min, followed by 93°C for 0.01 min, followed by 62°C for 0.2 min (30 cycles); 71°C for 3 min, followed by 62°C for 0.2 min, followed by 71°C for 3 min to complete final synthesis of DNA chains. One-tenth volume of the solution is analyzed by electrophoresis on 1 % agarose gels containing ethidium bromide in order to visualize amplified DNA products. Successful selective amplification of Ig H-chain sequences results in a DNA band of ca. 350 bp, viewed under u.v. illumination. Occasionally other irrelevent side products of the amplification reaction are observed as DNA bands of different size.

Reverse transcription and in vitro amplification of Ig L-chain mRNA is performed as above for H-chain mRNA, except that for the reverse transcription step the VH1FOR primer is replaced by the M/Cκ or VK1FOR oligonucleotide primer, and for the in vitro amplification step the VH1FOR and VH1BACK primers are replaced by VK1FOR and VK1BACK oligonucleotide primers. Successful amplification is monitored as described above for H-chain sequences, using agarose gel electrophoresis, and is indicated by the presence of a ca. 320 bp DNA band under u.v. illumination.

In both cases the amplified material is purified by extraction first with phenol/CHCl$_3$, and then CHCl$_3$. The material is finally precipitated with 2 volumes of ethanol at -20°C in the presence of 0.3 M NaOAc, pH 7.0, washed with 70% ethanol at the same temperature, and the DNA pellet air-dried.

4.3 Cloning of amplified Ig H- and L-chain cDNA sequences:

Amplified H- and L-chain cDNA (see example 4.2) is blunt-end cloned after preparing the ends of the DNA fragments by treatment with Klenow DNA polymerase and polynucleotide kinase.

DNA fragments prepared according to example 1.2 are dissolved in TE-buffer (20 μl), added separately to 17 μl of H$_2$O, 5 μl of mixed dNTP (1 mM each, N = A, T, G and C) and 5 μl of NT-buffer, and treated with 15 units (3 μl) of Klenow fragment DNA polymerase I (Boehringer) at room temperature for 30 min. The enzyme is inactivated by heating the solution at 65°C for 10 min, and the treated DNA samples are electrophoresed using TAE-buffer on 1% agarose gels containing ethidium bromide. DNA bands of ca. 350 bp for amplified H-chain DNA and ca. 320 bp for amplified L-chain DNA, visualised under u.v. illumination, are excised from the gel with a scalpel and purified on separate GENECLEAN™ columns (BIO 101 Inc.) according to the manufacturer's instructions. Each DNA is eluted in 30 μl of manufacturer's elution buffer, to which are added 7.5 μl of H$_2$O.

The purified H- and L-chain cDNA fragments (37.5 μl) are made up separately to 30 μl by the addition of 3 μl of PNK-buffer, 0.5 μl of dithiothreitol (0.1 M), 1 μl of spermidine (50 mM) and 3 μl of ATP ( 10 mM), and are each treated with 1 μl of T4 polynucleotide kinase (10 units; Pharmacia) at 37°C for 30 min. The reactions are terminated by the addition of 5 μl of 0.5 mM disodium-EDTA, pH 7.5, followed by 35 μl of TE-buffer and 10 μl of 3 M sodium acetate, pH 7.0, after which the DNA solutions are extracted with phenol/CHCl$_3$, CHCl$_3$, and precipitated with 2.5 volumes (v/v) of 95% ethanol. After centrifugation, supernatants are removed and DNA pellets dried and dissolved in 5 μl of TE-buffer.

DNA fragments are cloned in SmaI-digested, dephosphorylated BLUESCRIPT KS+ vector (Stratagene). Portions (1 μl) of the polynucleotide kinase-treated H- or L-chain cDNA fragments (1 ng) are separately ligated to 9 ng (1 μl) of prepared BLUESCRIPT™ vector in the presence of 1 μl of DNA ligase buffer, 1 μl of 10 x dithiothreitol (0.1 M), 0.5 μl of 20 x ATP (10 mM), 5.5 μl of H$_2$O, and 0.5 μl (200 units) of T4 DNA ligase (New England Biolabs.) overnight at 15°C.

DNA ligation products are transformed into competent cells of E. coli K12/BZ234 prepared using standard procedures. Ampicillin-resistant clones are picked and plasmid DNA is prepared. Clones with appropriate-sized DNA inserts can be identified by co-digestion of plasmid DNAs with EcoRI + XbaI, which cut the vector polymer sequence on opposite sides of the SmaI cloning site, and analysing DNA fragments by electrophoresis on 1 % agarose gels containing ethidium bromide.

4.4 Identification of functional Ig H- and L-chain gene rearrangement of hybridoma 25-57-1:

The nucleotide sequence of cloned plasmids with the correct sized DNA inserts are determined on double-stranded DNA templates with the SEQUENASE™ system using T3 and T7 oligonucleotide primers and the manufacturer's protocol (United States Biochemical). Several plasmid clones with identical sequences are obtained. Sequences of typical clones (25-LPCR1 and 25-HPCR1) are listed as SEQ ID NO:1 and SEQ ID NO:2 for L- and H-chain, respectively. Sequences shown include the oligonucleotide primers used for the selective in vitro amplification of Ig-related sequences, the position of DNA restriction sites located within the primers, and the location of complementarity-determining regions (CDRs) of the Ig L- and H-chain V-regions, deduced from comparison with the data base of murine Ig V-region sequences (Kabat et al., "Sequences of proteins of immunological interest", fourth edition, U.S. Dept. Health & Human Services, 1987).

The V-region rearrangement of 25-LPCR1 uses the J1 L-chain J-minigene joining exon (beginning at nucleotide position 286; SEQ ID NO:1 ) and includes a continuous open reading frame encoding a polypeptide sequence formed by V-J exon fusion, characteristic of a functional Ig L-chain gene rearrangement.

The V-region rearrangement of 25-HPCR1 uses the J$_H$3 H-chain J-minigene joining exon (beginning at nucleotide position 299; SEQ ID NO: 2) and includes a continuous open reading frame encoding a polypeptide sequence formed by V-D-J exon fusion, characteristic of a functional Ig H-chain gene rearrangement.

Occasional other DNA clones are obtained using 25-57-1 cell mRNA, none of which resemble functional Ig H- or L-chain V-region sequences. The sequences of at least three independent cloned plasmids containing H- and L-chain sequences are compared and shown to be identical, to reduce the possibility that sequences resulting from in vitro amplification contain point mutations in the V-region sequences.

### 4.5 Identification of functional Ig H- and L-chain gene rearrangement of hybridoma 26-29-5:

The nucleotide sequence of cloned plasmids with the correct sized DNA inserts are determined on double-stranded DNA templates with the SEQUENASE™ system using T3 and T7 oligonucleotide primers and the manufacturer's protocol (United States Biochemical). Several plasmid clones with identical sequences are obtained. Sequences of typical clones (26-LPCR1; 26-HPCR1 and 26-HPCR2) are listed as SEQ ID NO: 3 for the L-chain, SEQ ID NO:4 and SEQ ID NO:5 for the H-chains, respectively. Sequences shown include the oligonucleotide primers used for the selective in vitro amplification of Ig-related sequences, the position of DNA restriction sites located within the primers, and the location of complementarity-determining regions (CDRs) of the Ig L- and H-chain V-regions, deduced from comparison with the data base of murine Ig V-region sequences (Kabat et al., "Sequences of proteins of immunological interest", fourth edition, U.S. Dept. Health & Human Services, 1987).

The V-region rearrangement of 26-LPCR1 uses the J1 L-chain J-minigene joining exon (beginning at nucleotide position 304; SEQ ID NO:3) and includes a continuous open reading frame encoding a polypeptide sequence formed by V-J exon fusion, characteristic of a functional Ig L-chain gene rearrangement.

The V-region rearrangement of 26-HPCR1 uses the $J_H2$ H-chain J-minigene joining exon (beginning at nucleotide position 324; SEQ ID NO:4) and includes a continuous open reading frame encoding a polypeptide sequence formed by V-D-J exon fusion, characteristic of a functional Ig H-chain gene rearrangement.

The V-region rearrangement 26-HPCR2 uses the $J_H4$ H-chain J-minigene joining exon (beginning at nucleotide position 328; SEQ ID NO:5) and includes a continuous open reading frame encoding a polypeptide sequence format by V-D-J exon fusion, characteristic of a functional Ig H-chain gene rearrangement.

It is not decisively known whether the 26-HPCR1 and 26-HPCR2 rearrangements are contained within the same hybridoma cell. However, the H-chain rearrangement with the desired specificity is screened for by a functional test (see example 1.5).

Occasional other DNA clones are obtained using 26-69-5 cell mRNA, none of which resemble functional Ig H- or L-chain V-region sequences. The sequences of at least three independent cloned plasmids containing H- and L-chain sequences are compared and shown to be identical, to reduce the possibility that sequences resulting from in vitro amplification contain point mutations in the V-region sequences.

### Example 5. Construction of cloning vectors for DNA manipulation

### 5.1 Vector KS+exPvuII:

In order to facilitate cloning 25-LPCR1 and 26-LPCR1 sequences for expression in BLUESCRIPT KS+ plasmid vector (Stratagene), the (two) non-essential PvuII restriction sites are eliminated. BLUESCRIPT KS+ plasmid DNA (10 μg) is digested to completion with PvuII to generate two linear DNA fragments of ca. 2300 and 360 bp. The DNA fragments are separated by electrophoresis on 1 % agarose gels containing ethidium bromide, and purified using GENECLEAN™ (BIO 101 Inc.). This provides ca. 150 ng of purified 360 bp fragment and ca. 600 ng of 2300 bp fragment. The larger (2300 bp) fragment is treated with 20 units of calf intestinal alkaline phosphatase (CIAP; Boehringer). The CIAP-treated fragments are then extracted with phenol/$CHCl_3$, then $CHCl_3$, precipitated with 0.54 volumes of isopropanol in the presence of 0.3 M NaOAc, pH 7.0, and the DNA pellet is washed with 70% (v/v) ethanol at -20°C. The pellet is air-dried and dissolved in 6 μl of TE-buffer.

Oligonucleotide linkers (10 ng in 1 μl of $H_2O$) containing an NcoI DNA restriction site (pCCCATGGG; New England Biolabs.) are treated for 30 min at 37°C with 1 μl of T4 polynucleotide kinase (PL Biochemicals) in a solution containing 36.5 μl of $H_2O$, 5 μl of 10 x PNK-buffer, 0.5 μl of 0.1 M dithiothreitol, 1 μl of 50 mM spermidine and 5 μl of 10 mM ATP. The phosphorylated linkers are added to 1 μl (150 ng) of the 360 bp PvuII digested KS+ plasmid DNA fragment and 5 μl of CIAP-treated 2300 bp fragment (200 ng). The mixture of fragments is extracted with phenol/$CHCl_3$, then $CHCl_3$, and precipitated with ethanol in the presence of 0.3 M NaOAc, pH 7.0 at -20°C, washed with 70% (v/v) ethanol, air-dried and dissolved in 0.5 ml of $H_2O$. The plasmid DNA/linker mixture is ligated overnight at 4°C using 10,000 units/5 μl of T4 DNA ligase (New England Biolabs.) in a total volume of 1 ml of solution containing 100 μl of DNA ligase buffer, 100 μl of 0.1 M dithiothreitol, 50 μl of 20 mM ATP and 245 μl of $H_2O$. Ligation products are transformed into E. coli K12/BZ234, ampicillin-resistant colonies selected and plasmid DNA preparations made using standard procedures (Sambrook et al., op. cit., Section 1.82-84 & Section 1.25-28). Plasmids having acquired NcoI-linkers ligated at both of the junctions between the 360 bp and 2300 bp DNA fragments are detected by DNA restriction analysis using NcoI.

The NcoI restriction sites are removed from one such plasmid by digesting to termination with NcoI, treating a sample of the digested DNA with Klenow DNA polymerase to generate blunt-end fragments and eliminate the NcoI "sticky-ends", and religation of the fragments using T4 DNA ligase, all using standard procedures as described above and in example 4.3.

The final plasmid, designated KS+exPvuII, contains NcoI linkers at the original locations of the PvuII restriction sites in the KS+ vector, in which the NcoI restriction sites have been eliminated using Klenow DNA polymerase. The relative orientation of the 360 bp and 2300 bp KS+ DNA fragments and the polylinker DNA cloning sites remain the same as in the BLUESCRIPT™ KS+ vector.

## 5.2 Vectors KS+VHX-Vec and KS+VKX-Vec:

The vectors M13-VHPCR1 and M13-VKPCR1 (Orlandi et al., Proc. Natl. Acad. Sci. USA 86, 3833-3837, 1989, made available by The Medical Research Council, 20 Park Crescent, London W1N 4AL) contain 817 bp and 648 bp BamHI/HindIII restriction fragments, respectively, capable of accepting Ig H- and L-chain V-region DNA sequences amplified from F5-444 cell RNA using oligonucleotides as described in examples 4.2 and 4.3. These BamHI/HindIII vector DNA fragments contain mouse Ig promoters, leader peptide exons and functional rearranged V-D-J and V-J V-region exons.

This DNA is transformed into E. coli K12/TG1, and plasmid DNAs are prepared using standard procedures: Each bacteriophage DNA preparation is digested separately to termination using BamHI + HindIII, and the smaller BamHI/HindIII fragment isolated in each case (817 bp for M13-VHPVR1, and 648 bp for M13-VKPCR1) by electrophoresis on 0.8% agarose gels containing ethidium bromide. DNA bands of the correct size, visualized under u.v. illumination, are excised from the gel and DNA recovered by electroelution from the agarose followed by phenol/CHCl$_3$ extraction and precipitation with isopropanol. DNA pellets are dried and dissolved separately in 10 μl of TE-buffer.

### 5.2.1 Vector KS+VHX-Vec:

In M13-VHPCR1 the BamHI/HindIII segment contains unique internal DNA restriction sites for enzymes PstI and BstEII, allowing removal of the "irrelevant" Ig H-chain V-region and replacement by a V-region sequence such as that located between PstI and BstEII restriction sites of 25-HPCR1, 26-HPCR1 and 26-HPCR2. The exact location of PstI and BstEII sites in the DNA amplification primers VH1FOR and VH1BACK ensure that the H-chain polypeptide-coding sequence is maintained in the correct translational frame with the surrounding DNA sequences, facilitating expression of the V-region protein.

The 817 bp M13-VHPCR1 DNA fragment is cloned after ligation to BLUESCRIPT™ KS+ vector. The vector is prepared for use by digestion with restriction enzymes BamHI+ HindIII, followed by extraction with phenol-/CHCl$_3$ and CHCl$_3$, ethanol precipitation, centrifugation, washing with 70% ethanol, drying the DNA pellet and dissolving in TE-buffer. Ligation is carried out for 6 h at 14°C in 15 μl of solution containing 2 μl of BamHI + HindIII-treated vector DNA (40 μg), 2.5 μl of M13-VHPCR1 DNA fragment (2.5 μl), 1.5 μl of 10 mM ATP, 1.5 μl of 0.1 M dithiothreitol, 1.5 μl of DNA ligase buffer, 4.5 μl of TE-buffer and 1.5 μl (600 units) of T4 DNA ligase (New England Biolabs.). Ligation products are transformed into E. coli K12/TG1, ampicillin-resistant colonies selected and plasmid DNAs prepared using standard procedures (Sambrook et al., op. cit., Section 1.82-84 & 1.25-28). A clone is selected containing a recombinant plasmid with a BamHI/HindIII insert DNA fragment of 817 bp, and its sequence confirmed as corresponding to the expected fragment derived from M13-VHPCR1 using the SEQUENASE™ system with T3/T7 oligonucleotide primers, using conditions provided by the manufacturer (United States Biochemical). This plasmid is referred to as KS+VH-Vec.

KS+VH-Vec is further modified by the introduction of a second XbaI DNA restriction site in addition to the one originally derived from the BLUESCRIPT™ KS+polylinker. KS+VH-Vec DNA is digested to termination with restriction endonuclease HinDII, and the fragments are dephosphorylated with calf intestinal alkaline phosphatase using standard procedures (see example 2.1). After treatment the DNA is dissolved in TE-buffer at a concentration of 120 ng/ml. After dissolution, the DNA fragments (0.5 μl) are added to 1 μl of XbaI oligonucleotide linkers (pCTCTAGAG, 500 ng/μl; New England Biolabs.), 1.5 μl of 10 mM ATP, 1.5 μl of 0.1 M dithiothreitol, 1.5 μl of DNA ligase buffer and 7.5 μl of TE-buffer, and ligated overnight at 14°C using 1.5 μl (600 units) of T4 DNA ligase (New England Biolabs.). Following ligation, the mixture is used to transform E. coli K12/TG1, ampicillin-resistant colonies are selected and plasmid DNA preparations mammal using standard procedures, as described above (Sambrook et al., op. cit., Section 1.82-84 & 1.25-28). Plasmids having acquired the XbaI-linker sequence are detected by digestion using restriction endonuclease XbaI. One plasmid is selected and its nucleotide sequence confirmed as having an additional XbaI-linker sequence incorporated at the original HindII restriction site in the BLUESCRIPT™ KS+ sequence. The plasmid is referred to as SK+VHX-Vec.

5.2.2 Vector KS+VKX-Vec:

In M13-VKPCR1, the BamHI/HindIII segment contains unique internal DNA restriction sites for enzymes PvuII and BclI, allowing removal of the "irrelevant" Ig L-chain V-region and replacement by a V-region sequence such as that located between PvuII and BglII restriction sites of 25-LPCR1 or 26-LPCR1. The exact location of PvuII and BglII sites in the DNA amplification primers VK1FOR and VK1BACK ensures that the L-chain polypeptide-coding sequence is maintained in the correct translational frame with the surrounding DNA sequences, facilitating expression of the V-region protein. Since the DNA restriction enzyme BclI is sensitive to Dam+ methylation, an appropriate Dam⁻ host strain, e.g. E. coli K12/BZ103, K12/E3225 or K12/R832, must be used for growth of phage and plasmids where it is intended, as here, to utilize the BclI restriction site for cloning purposes.

The 648 bp BamHI/HindIII DNA fragment of M13-VKPCR1 is cloned in a similar manner to that of M13-VHPCR1 (example 5.2.1) by ligation to BamHI/HindIII-digested KS+exPvuII vector (described in example 5.1). After transformation colonies are selected, and one containing a recombinant plasmid with a BamHI/HinDIII insert fragment of 648 bp is analyzed and its sequence confirmed as corresponding to the expected fragment derived from M13-VKPCR1 using the SEQUENASE™ system with T3/T7 oligonucleotide primers as described above. This plasmid is referred to as KS+VK-Vec.

The KS+VK-Vec vector is further modified by introduction of an XbaI-linker at the HindII restriction site in the BLUESCRIPT™ KS+polylinker sequence, and its sequence confirmed in a similar manner to that described above for KS+VHX-Vec (example 5.2.1). Plasmid DNA is transformed into E. coli K12/R832 (Dam⁻) in order to maintain its BclI restriction site in the unmethylated state for cloning purposes. The final plasmid vector is referred to as KS+VKX-Vec.

Example 6. Construction of a vector for Ig H-chain gene expression in myeloma cells

6.1 Isolation of a DNA fragment encoding a human γ1 H-chain:

A human DNA library is constructed in the bacteriophage λ vector Charon 4a by limited digestion of human fetal liver DNA with restriction endonucleases HaeIII and AluI using published procedures (Lawn et al., Cell 15, 1157, 1978). Approximately $1 \times 10^6$ independent recombinant phages are plated on E. coli K12/803 and screened for the presence of human Ig H-chain DNA sequences using an homologous murine Ig H-chain DNA probe.

A nick-translated ³²P-labelled mouse IgG H-chain DNA probe corresponding to the XbaI/HhaI fragment of the mouse Ig γ2b gene locus is used to screen recombinant phage as described previously (Takahashi et al., Cell 29, 671-679, 1982). One DNA clone analyzed (#95/4) contains a 7 kb HindIII DNA segment encompassing the human IgG1 constant region exons CH1, hinge region, CH2 and CH3, as determined by restriction mapping and by nucleotide sequence analysis. The portion of clone #95/4 that is sequenced corresponds to the published human IgG1 gene sequence (EMBL data base sequence entry HUMIGCC4) which starts from one of the terminal (HindIII) restriction sites of the 7 kb HindIII segment (Ellison et al., Nucleic Acids Res. 10, 4071-4079, 1982).

The 7 kb HindIII DNA fragment from clone #95/4 containing the human IgG1 constant region exons is subcloned into the HindIII site of dephosphorylated BLUESCRIPT™ KS+ vector and transformed into E. coli K12/TG1. Ampicillin-resistant colonies are picked, plasmid DNAs prepared and those containing the subcloned 7 kb DNA fragment isolated using standard procedures (Sambrook et al., op. cit., Section 1.82-84 & 1.25-28). Plasmid DNA from one clone is identified in which the HindIII site located 5′ of the human IgG1 CH1 exon is oriented next to the XbaI DNA restriction site of the polylinker of BLUESCRIPT™ KS+. The plasmid is designated γ1-KS+.

The entire 7 kb DNA insert of γ1-KS+ containing the human IgG1 coding sequences is recovered by electrophoresis on 1 % agarose gels containing ethidium bromide after digestion of γI-KS+ using EcoRI/XbaI which cleaves the KS+ polylinker on both sides of the human DNA insert (neither enzyme cleaves the 7 kb HindIII insert DNA fragment of yI-KS+). The DNA fragment is electroeluted, extracted with phenol/CHCl₃, then CHCl₃, and recovered by ethanol precipitation/centrifugation using standard procedures. This DNA is referred to as Fragment 1. The DNA is dissolved in TE-buffer and stored at -20°C.

6.2 Cloning of a DNA fragment containing the murine Ig H-chain transcriptional enhancer:

A 686 bp XbaI/EcoRI DNA fragment corresponding to nucleotides 2877-3563 of the murine Ig H-chain gene locus (GENBANK data base entry MUSSIGCD07) containing the murine Ig H-chain transcriptional enhancer is cloned in EcoRI/XbaI-gested BLUESCRIPT™ KS+ vector and its sequence verified using the SEQUENASE™ system with T3 and T7 olignonucleotide primers. The plasmid is designated pDA24/3. The insert DNA fragment of pDA24/3 is isolated after digestion with EcoRI/XBaI, and recovered after electrophoresis on 1% agarose gels containing ethidium bromide using the same procedure used for Fragment 1. The DNA is dissolved in TE-buffer and stored at -20°C. This DNA is referred to as Fragment 2.

### 6.3 Assembly of plasmid vector Huγl-EH-gpt:

DNA Fragment 1 and Fragment 2 (examples 6.1 and 6.2) are cloned by coligation into vector pSV2gpt (Southern & Berg, J. Mol. App. Genet. 1, 327, 1982), linearised by digestion using EcoR1. The DNA ligation mixture contains digested pSV2gpt vector DNA (50 ng in 1 μl of TE-buffer), Fragment 1 (70 ng in 1.3 μl of TE-buffer), Fragment 2 (7 ng in 1.0 μl of TE-buffer), 1.5 μl of 10 mM ATP, 1.5 μl of 0.1 M dithiothreitol, 1.5 μl of DNA ligase buffer, 5.7 μl of TE-buffer and 1.5 μl of T4 DNA ligase (600 units; New England Biolabs.). Ligation is performed overnight at 14°C. After transformation into E. coli K12/TG1, ampicillin-resistant recombinants are identified and plasmid DNAs prepared using standard-procedures (Sambrook et al., op. cit., Section 1.82-84 & 1.25-28). DNA clones are selected based on the results of restriction analysis using the enzymes EcoRI, BamHI, HindIII, PvuII and XbaI. A plasmid with the appropriate DNA restriction properties is selected and designated Huγl-EH-gpt. The plasmid contains pSV2gpt sequences and the mouse Ig H-chain enhancer and human IgGI constant region exons separated by a unique XbaI cleavage site.

### Example 7. Construction of a vector for Ig L-chain gene expression in myeloma cells

### 7.1 Isolation of a DNA fragment encoding the human Cκ L-chain constant region exon:

A nick-translated $^{32}$P-labelled murine Ig germline L-chain J-region genomic DNA probe is prepared, corresponding to a ca. 2240 bp HindIII/XbaI segment of Balb/c mouse liver DNA (nucleotide positions 659-2900 of the published germline Ig L-chain locus; Max et al., Proc. Natl. Acad. Sci. USA 76, 3450-3454, 1979; EMBL data base sequence entry MUSIGKJC2). The probe is used to screen a human recombinant λ phage DNA library for segments containing homologous human Cκ L-chain DNA sequences, using a similar procedure to that described for Ig H-chain sequences (example 6.1). A positively-hybridizing recombinant phage is selected and DNA prepared using standard procedures.

A DNA fragment is subcloned from the cross-hybridizing recombinant phage, corresponding to a ca. 2.5 kb BalI/EcoRI fragment containing the human Ig Cκ constant region exon (Hieter et al., Cell 22, 197-207, 1980). The BalI DNA restriction site and Cκ coding region are indicated in the EMBL data base sequence entry HUMIGKC3; BalI DNA cleavage site = nucleotide position 31; Cκ coding region = nucleotide position 334-656. The cloning vector used is SmaI/EcoRI-digested BLUESCRIPT™ KS+, and the methods used are standard cloning procedures (Sambrook et al., op. cit., Section 1.63-70). A recombinant plasmid with the desired DNA restriction characteristics is selected, and its structure confirmed by partially sequencing using the SEQUENASE™ system with T3 and T7 oligonucleotide primers according to the manufacturer's protocol. The plasmid is designated pDA27.

pDA27 contains two DNA restriction sites for the enzyme XbaI; one derived from the KS+ polylinker, the second being a site in the human Cκ DNA insert of pDA27 located ca. 1 kb from the EcoRI site. In order to facilitate subsequent use in the construction of expression vectors the latter DNA restriction site is eliminated as follows: pDA27 DNA (30 μg) is partially digested with 4 μl of XbaI (1 unit/μl; Boehringer) for 45 min at 37°C. The DNA is then extracted with phenol/CHCl$_3$, CHCl$_3$, ethanol precipitated and dissolved in 82 μl of distilled H$_2$O to which is added 10 μl of NT-buffer, 4 μl of mixed dNTPs (2 mM each; N = A, T, G and C). Klenow fragment DNA polymerase (4 μl; 4.9 units/μl; Boehringer) is added and the mixture incubated at room temperature for 30 min, after which the enzyme is heat-inactivated by heating at 65°C for 5 min. Partial digestion generates DNA fragments of ca. 5.5 kb which are separated from terminal digestion products by electrophoresis on a 0.8% agarose gel containing ethidium bromide. After electrophoresis the 5.5 kb DNA band is electroeluted from the agarose gel and recovered by phenol/chloroform extraction and ethanol precipitation as described above. The DNA pellet is dissolved in 20 μl of TE-buffer (yield approx. 40 ng). One-half of the material is religated, transformed into E. coli K12/BZ234, and ampicillin-resistant colonies are selected. Plasmid DNA is prepared from appropriate clones and digested with a combination of EcoRI + XbaI. Plasmids with the correct XbaI site eliminated generate two EcoRI/XbaI DNA restriction fragments; one of ca. 3 kb (containing KS+ vector sequences) and one of 2.5 kb (human Cκ-containing fragment). One such plasmid is selected and referred to as pDA28.

The 2.5 kb human DNA fragment of pDA28, now containing an XbaI DNA restriction site (derived from the KS+ polylinker) located 5'of the human Cκ-coding region, is recovered from the plasmid by digestion with

EcoRI/XbaI, and purified by electrophoresis on 0.8% agarose gels followed by extraction with phenol/CHCl₃, then CHCl₃, ethanol precipitation, drying and dissolution in TE-buffer. The DNA is referred to as <u>Fragment 3</u>.

## 7.2 Assembly of plasmid vector HuCκ-EH-neo:

DNA <u>Fragment 2</u> (ca. 700 bp EcoRI/XbaI DNA fragment containing the murine Ig H-chain transcriptional enhancer, example 6.2) and <u>Fragment 3</u> (ca. 2.5 kb EcoRI/XbaI DNA fragment containing the human Cκ coding region; example 7.1) are cloned by coligation into vector pSV2neo (Southern & Berg, J. Mol. App. Genet. 1, 327, 1982), linearized by digestion using EcoRI. DNA is ligated overnight at 14°C in a mixture containing 20 ng of EcoRI-digested pSV2neo DNA (1 μl), 15 ng of DNA <u>Fragment 2</u> (1 μl), 25 ng of DNA <u>Fragment 3</u> (0.5 μl), 2 μl of DNA ligase buffer, 2 μl of 10 mM ATP, 2 μl of 0.1 M dithiothreitol and 10 μl of H₂O, using 1 μl of T4 DNA ligase (400 units/μl; New England Biolabs.). The ligation mixture is transformed into <u>E. coli</u> K12/803, ampicillin-resistant colonies are selected, and plasmid DNAs prepared using standard procedures (Sambrook et al., op. cit., Section 1.82-84 & 1.25-28). Plasmids are analyzed by digestion with EcoRI/XbaI and Pst1, and one with the desired orientation of DNA fragments selected, referred to as HuCκ-EH-neo.

## Example 8. <u>Vectors for expression of chimeric mouse/human monoclonal antibody in myeloma cells</u>

### 8.1 Generation of the chimeric Ig H-chain expression vector 25-Huγ1H1:

Vector KS+VHX-Vec (7.5 μg; example 5.2.1) is digested to termination using restriction endonucleases BstEII/XbaI, and the DNA fragments fractionated on a 0.8% agarose gel containing ethidium bromide. Digestion releases the "irrelevant" V-region gene segment (ca. 330 bp) and a ca. 3.3 kb BstEII/XbaI vector DNA fragment, which is excised from the gel, recovered by electroelution, extracted with phenol/CHCl₃, then CHCl₃, and precipitated with ethanol. The DNA pellet obtained after centrifugation is washed in 70% ethanol at -20°C and dissolved in distilled H₂O.

Plasmid 25-HPCR1 (ca. 30 μg; example 1.4) is digested to completion with restriction endonucleases BstEII/PstI, releasing a ca. 330 bp DNA fragment containing the cloned 25-57-1 Ig H-chain V-region (SEQ ID NO:2). The fragment is separated from vector sequences by electrophoresis on a 1 % agarose gel, recovered by fractionation using GENECLEAN (BIO 101 Inc.) using the procedure provided by the manufacturer. DNA is precipitated at -20°C with 2.5 volumes of 95% ethanol in the presence of 0.3 M NaOAc, pH 7.0, washed using 70% ethanol at the same temperature, and dissolved in TE-buffer. The yield is ca. 400 ng of DNA.

The isolated ca. 3.3 kb vector DNA fragment (100 ng in 2 μl of H₂O) isolated from KS+VHX-Vec as described above is ligated overnight at 4°C together with the isolated ca. 330 bp 25-57-1 Ig H-chain V-region DNA fragment (20 ng in 1 μl of TE-buffer) in the presence of 1 μl of DNA ligase buffer, 1 μl of 0.1 M dithiothreitol, 0.5 μl of 20 mM ATP, 3.5 μl of H₂O and 1 μl of T4 DNA ligase (400 units/μl; New England Biolabs.). DNA ligation products are transformed into <u>E. coli</u> K12/TG1, ampicillin-resistant colonies selected, and plasmid DNA clones identified. Several clones are identified with the expected DNA restriction properties, and one clone containing the 25-57-1 Ig H-chain V-region sequenced using the SEQUENASE™ system with T3/T7 oligonucleotide primer, using the manufacturer's protocol. The DNA clone is referred to as clone 25-M13VHPCR1.

25-M13VHPCR1 contains the 25-57-1 Ig H-chain V-region adapted for expression by incorporation into the M13-VHPCR1 cassette (described in example 5.2). The adapted H-chain V-region exon, plus the H-chain leader peptide exon and promoter element derived from the M13-VHPCR1 cassette, are released together from clone 25-M13VHPCR1 on a ca. 830 bp DNA fragment by partial digestion with XbaI (since the H-chain V-region contains an internal XbaI DNA restriction site within the coding sequence). The partial XbaI insert DNA fragment is separated from the larger vector-containing DNA fragment and the smaller terminal XbaI DNA restriction fragment by electrophoresis on a 0.8% agarose gel, purified by phenol/CHCl₃ extraction and ethanol precipitation by standard procedures, and dissolved in TE-buffer. The DNA fragment is ligated to plasmid vector Huγ1-EH-gpt (example 6.3) as follows: 200 ng of XbaI-digested, dephosphorylated Huγ1-EH-gpt (in 2 μl of TE-buffer) and 10 ng of isolated DNA fragment (in 0.5 μl of TE-buffer) are added to 2 μl of DNA ligase buffer, 2 μl of 0.1 M dithiothreitol, 1 μl of 20 mM ATP and 10.5 μl of H₂O. DNA ligation is performed overnight at 4°C using 2 μl of T4 DNA ligase (800 units; New England Biolabs.). DNA ligation products are transformed into <u>E. coli</u> K12/BZ234, ampicillin-resistant colonies selected and plasmid DNAs prepared using standard procedures (Sambrook et al., op. cit., Section 1.82-84 & 1.25-28). Plasmids containing the inserted ca. 830 bp XbaI DNA fragment in the required orientation for expression are detected by using BstEII. Plasmid DNA is prepared from one DNA clone, referred to as 25-Huγ1H1.

8.2 Generation of the chimeric Ig L-chain expression vector 25-HuCκL1:

Vector KS+VKX-Vec (60 μg; example 5.2.2) is digested to termination using restriction endonucleases BclI/PvuII, dephosphorylated by treatment with calf intestinal alkaline phosphatase using a standard procedure (see example 5.1) and the DNA fragments fractionated on a 0.8% agarose gel containing ethidium bromide using TBE-buffer. Digestion releases the "irrelevant" V-region gene segment (ca. 300 bp) and a ca. 3.3 kb BclI/PvuII vector DNA fragment, which is excised from the gel, recovered by electroelution, extracted with phenol/CHCl$_3$, then CHCl$_3$, and precipitated with isopropanol. The DNA pellet obtained by centrifugation is washed in 70% ethanol at -20°C and dissolved in 100 μl of TE-buffer.

Plasmid 25-LPCR1 (example 4.4) is digested to completion with restriction endonucleases BglII/PvuII, releasing a ca. 320 bp DNA fragment containing the cloned 25-57-1 Ig L-chain V-region (SEQ ID NO:1). The fragment is separated from vector sequences by electrophoresis on a 0.8% agarose gel, recovered by electroelution, extracted with phenol / CHCl$_3$, then CHCl$_3$, precipitated at -20°C with 2.5 volumes of 95% ethanol in the presence of 0.3 M NaOAc, pH 7.0, washed using 70% ethanol at the same temperature, and dissolved in TE-buffer.

The ca. 3.3 kb dephosphorylated vector DNA fragment (75 ng in 2 μl TE-buffer), isolated from KS+VKX-Vec as described above, is ligated together with the isolated ca. 320 bp 25-LPCR1 Ig L-chain V-region DNA fragment (30 ng in 1 μl of TE-buffer) in the presence of 2 μl of 0.1 M dithiothreitol, 1 μl of 20 mM ATP, 10 μl of TE-buffer, 2 μl of DNA ligase buffer and 2 μl (800 units; New England Biolabs.) of T4 DNA ligase. DNA ligation products are transformed into E. coli K12/803, ampicillin-resistant colonies selected, plasmid DNA isolated and clones identified by restriction analysis using XbaI, and sequence analysis performed using the SEQUENASE™ system with T3 and T7 oligonucleotide primers according to the manufacturer's protocol. One clone is selected. The DNA clone is referred to as clone 25-M13VLPCR1.

Clone 25-M13VLPCR1 contains the 25-LPCR1 Ig L-chain V-region adapted for expression by incorporation into the M13-VKPCR1 cassette (described in example 5.2). The adapted L-chain V-region exon, plus the L-chain leader peptide exon and promoter element derived from the M13-VKPCR1 cassette, are released together from clone 25-M13VLOCR1 on a ca. 630 bp DNA fragment by digestion with XbaI. This smaller XbaI DNA fragment is separated from the larger vector-containing DNA fragment by electrophoresis on a 0.8% agarose gel, purified by phenol / CHCl$_3$ extraction and ethanol precipitation by standard procedures, and dissolved in TE-buffer. The DNA fragment is ligated to plasmid vector HuCκ-EH-neo (example 7.2) as follows: 50 ng of XbaI-digested, dephosphorylated HuCκ-EH-neo (in 10 μl of TE-buffer) and 20 ng of isolated DNA fragment (in 2 μl of TE-buffer) are added to 2 μl of DNA ligase buffer, 2 μl of 0.1 M dithiothreitol, 2 μl of 10 mM ATP, 1.5 μl of H$_2$O, and 2 μl of T4 DNA ligase (400 units; New England Biolabs.). Ligation is performed overnight at 14°C. DNA ligation products are transformed into E. coli K12/803, ampicillin-resistant colonies selected and plasmid DNAs prepared using standard procedures (Sambrook et al., op. cit., Section 1.82-84 & 1.25-28). Plasmids containing the inserted ca. 630 bp XbaI DNA fragment in the required orientation for expression are detected by digestion using restriction endonuclease PstI. DNA from one such plasmid is prepared, and is referred to as 25-HuCκL1.

8.3 Generation of the chimeric Ig H-chain expression vectors 26-Huγ1H1 and 26-Huγ1H2:

Vector KS+VHX-Vec (7.5 μg; example 5.2.1) is digested to termination using restriction endonucleases BstEII/XbaI, and the DNA fragments fractionated on a 0.8% agarose gel containing ethidium bromide. Digestion releases the "irrelevant" V-region gene segment (ca. 330 bp) and a ca. 3.3 kb BstEII/XbaI vector DNA fragment, which is excised from the gel, recovered by electroelution, extracted with phenol/CHCl$_3$, then CHCl$_3$, and precipitated with ethanol. The DNA pellet obtained after centrifugation is washed in 70% ethanol at -20°C and dissolved in distilled H$_2$O.

Plasmid 26-HPCR1 (example 4.5) is digested to completion with restriction endonucleases BstEII/PstI, releasing a ca. 330 bp DNA fragment containing the cloned 26-HPCR1 Ig H-chain V-region (SEQ ID NO: 4). The fragment is separated from vector sequences by electrophoresis on a 1 % agarose gel, recovered by fractionation using GENECLEAN™ (BIO 101 Inc.) using the procedure provided by the manufacturer. DNA is precipitated at -20°C with 2.5 volumes of 95% ethanol in the presence of 0.3 M NaOAc, pH 7.0, washed using 70% ethanol at the same temperature, and dissolved in TE-buffer.

The isolated ca. 3.3 kb vector DNA fragment (18 ng in 2 μl of H$_2$O) isolated from KS+VHX-Vec as described above is ligated overnight at 4°C together with the isolated ca. 330 bp 26-HPCR1 Ig H-chain V-region DNA fragment (2 ng in 3 μl of TE-buffer) in the presence of 2 μl of DNA ligase buffer, 2 μl of 0.1 M dithiothreitol, 1.0 μl of 20 mM ATP, 9.5 μl of H$_2$O and 0.5 μl of T4 DNA ligase (400 units/μl; New England Biolabs.). DNA ligation products are transformed into E. coli K12/803, ampicillin-resistant colonies selected, and plasmid DNA clones identified. Several clones are identified with the expected DNA restriction properties, and one clone containing the 26-HPCR1 Ig H-chain V-region sequenced using the SEQUENASE™ system with T3/T7 oligonucleotide primer, using the manufacturer's protocol. The DNA clone is referred to as clone 26-M13VHPCR1.

Clone 26-M13VHPCR1 contains the 26-HPCR1 Ig H-chain V-region adapted for expression by incorporation into the M13-VHPCR1 cassette (described in example 5.2). The adapted H-chain V-region exon, plus the H-chain leader peptide exon and promoter element derived from the M13-VHPCR1 cassette, are released together from clone 26-M13VHPCR1 on a ca. 830 bp DNA fragment by digestion with XbaI. The XbaI fragment is separated from the larger vector-containing DNA fragment by electrophoresis on a 0.8% agarose gel, purified by phenol/CHCl$_3$ extraction and ethanol precipitation by standard procedures, and dissolved in TE-buffer. The DNA fragment is ligated to plasmid vector Huγ1-EH-gpt (example 6.3) as follows: 200 ng of XbaI-digested, dephosphorylated Huγ1-EH-gpt (in 2 μl of TE-buffer) and 10 ng of isolated DNA fragment (in 0.5 μl of TE-buffer) are added to 2 μl of DNA ligase buffer, 2 μl of 0.1 M dithiothreitol, 1 μl of 20 mM ATP and 10.5 μl of H$_2$O. DNA ligation is performed overnight at 4°C using 2 μl of T4 DNA ligase (800 units; New England Biolabs.). DNA ligation products are transformed into E. coli K12/803, ampicillin-resistant colonies selected and plasmid DNAs prepared using standard procedures (Sambrook et al., op. cit., Section 1.82-84 & 1.25-28). Plasmids containing the inserted ca. 830 bp XbaI DNA fragment in the required orientation for expression are detected by using BstEII. Plasmid DNA is prepared from one DNA clone, referred to as 26-Huγ1H1.

A similar procedure is used to generate an expression vector for the 26-HPCR2 H-chain. Plasmid 26-HPCR2 (example 4.5) is digested to completion with restriction endonucleases BstEII and partially with PstI (since the 26-HPCR2 V-region contains two internal restriction sites for PstI), releasing a ca. 330 bp DNA fragment containing the intact 26-HPCR2 Ig H-chain V-region (SEQ ID NO: 5). The fragment is separated from vector sequences by electrophoresis on a 1% agarose gel, recovered by fractionation using GENECLEAN™ (BIO 101 Inc.) using the procedure provided by the manufacturer. DNA is precipitated at -20°C with 2.5 volumes of 95% ethanol in the presence of 0.3 M NaOAc, pH 7.0, washed using 70% ethanol at the same temperature, and dissolved in TE-buffer.

The isolated ca. 3.3 kb vector DNA fragment (100 ng in 2 μl of H$_2$O) isolated from KS+VHX-Vec as described above is ligated overnight at 4°C together with the isolated ca. 330 bp 26-HPCR2 Ig H-chain V-region DNA fragment (10 ng in 1 μl of TE-buffer) in the presence of 1 μl of DNA ligase buffer, 1 μl of 0.1 M dithiothreitol, 0.5 μl of 20 mM ATP, 3.5 μl of H$_2$O and 1 μl of T4 DNA ligase (400 units/μl; New England Biolabs.). DNA ligation products are transformed into E. coli K12/803, ampicillin-resistant colonies selected, and plasmid DNA clones identified. Several clones are identified with the expected DNA restriction properties, and one clone containing the 26-HPCR2 Ig H-chain V-region sequenced using the SEQUENASE™ system with T3/T7 oligonucleotide primer, using the manufacturer's protocol. The DNA clone is referred to as clone 26-M13VHPCR2.

Clone 26-M13VHPCR2 contains the 26-HPCR2 Ig H-chain V-region adapted for expression by incorporation into the M13-VHPCR1 cassette (described in example 5.2). The adapted H-chain V-region exon, plus the H-chain leader peptide exon and promoter element derived from the M13-VHPCR1 cassette, are released together from clone 26-M13VHPCR2 on a ca. 830 bp DNA fragment by digestion with XbaI. The XbaI fragment is separated from the larger vector-containing DNA fragment by electrophoresis on a 0.8% agarose gel, purified by phenol/CHCl$_3$ extraction and ethanol precipitation by standard procedures, and dissolved in TE-buffer. The DNA fragment is ligated to plasmid vector Huγ1-EH-gpt (example 6.3) as follows: 200 ng of XbaI-digested, dephosphorylated Huγ1-EH-gpt (in 2 μl of TE-buffer) and 10 ng of isolated DNA fragment (in 0.5 μl of TE-buffer) are added to 2 μl of DNA ligase buffer, 2 μl of 0.1 M dithiothreitol, 1 μl of 20 mM ATP and 10.5 μl of H$_2$O. DNA ligation is performed overnight at 4°C using 2 μl of T4 DNA ligase (800 units; New England Biolabs.). DNA ligation products are transformed into E. coli K12/803, ampicillin-resistant colonies selected and plasmid DNAs prepared using standard procedures (Sambrook et al., op. cit., Section 1.82-84 & 1.25-28). Plasmids containing the inserted ca. 830 bp XbaI DNA fragment in the required orientation for expression are detected by using BstEII. Plasmid DNA is prepared from one DNA clone, referred to as 26-Huγ1H2.

8.4 Generation of the chimeric Ig L-chain expression vector 26-HuCκL1:

Vector KS+VKX-Vec (60 μg; example 5.2.2) is digested to termination using restriction endonucleases BclI/PvuII, dephosphorylated by treatment with calf intestinal alkaline phosphatase using a standard procedure (see example 5.1) and the DNA fragments fractionated on a 0.8% agarose gel containing ethidium bromide using TBE-buffer. Digestion releases the "irrelevant" V-region gene segment (ca. 300 bp) and a ca. 3.3 kb BclII/PvuII vector DNA fragment, which is excised from the gel, recovered by electroelution, extracted with phenol/CHCl$_3$, then CHCl$_3$, and precipitated with isopropanol. The DNA pellet obtained by centrifugation is washed in 70% ethanol at -20°C and dissolved in 100 μl of TE-buffer.

Plasmid 26-LPCR1 (example 4.5) is digested to completion with restriction endonucleases BglII/PvuII, releasing a ca. 320 bp DNA fragment containing the cloned 26-LPCR1 Ig L-chain V-region (SEQ ID NO: 3). The fragment is separated from vector sequences by electrophoresis on a 0.8% agarose gel, recovered by elec-troelution, extracted with phenol/CHCl$_3$, then CHCl$_3$, precipitated at -20°C with 2.5 volumes of 95% ethanol in the presence of 0.3 M NaOAc, pH 7.0, washed using 70% ethanol at the same temperature, and dissolved in TE-buffer

The ca. 3.3 kb dephosphorylated vector DNA fragment (9 ng in 1 µl TE-buffer), isolated from KS+VKX-Vec as described above, is ligated together with the isolated ca. 320 bp 26-LPCR1 Ig Lchain V-region DNA fragment (1 ng in 1 µl of TE-buffer) in the presence of 1 µl of 0.1 M dithiothreitol, 1 µl of 0.5 mM ATP, 5 µl of H$_2$O, 1 µl of DNA ligase buffer and 0.5 µl (800 units; New England Biolabs.) of T4 DNA ligase. DNA ligation products are transformed into E. coli K12/BZ234, ampicillin-resistant colonies selected, plasmid DNA isolated and clones identified by restriction analysis using XbaI, and sequence analysis performed using the SEQUENASE™ system with T3 and T7 oligonucleotide primers according to the manufacturer's protocol. One clone is selected. The DNA clone is referred to as clone 26-M13VLPCR1.

Clone 26-M13VLPCR1 contains the 26-LPCR1 Ig L-chain V-region adapted for expression by incorporation into the M13-VKPCR1 cassette (described in example 5.2). The adapted L-chain V-region exon, plus the L-chain leader peptide exon and promoter element derived from the M13-VKPCR1 cassette, are released together from clone 26-M13VLPCR1 on a ca. 630 bp DNA fragment by digestion with XbaI. This smaller XbaI DNA fragment is separated from the larger vector-containing DNA fragment by electrophoresis on a 0.8% agarose gel, purified by phenol/CHCl$_3$ extraction and ethanol precipitation by standard procedures, and dissolved in TE-buffer. The DNA fragment is ligated to plasmid vector HuCκ-EH-neo (example 7.2) as follows: 100 ng of XbaI-digested, dephosphorylated HuCκ-EH-neo (in 1 µl of TE-buffer) and 20 ng of isolated DNA fragment (in 4 µl of TE-buffer) are added to 1 µl of DNA ligase buffer, 1 µl of 0.1 M dithiothreitol, 0.5 µl of 20 mM ATP, 1.5 µl of H$_2$O, and 1 µl of T4 DNA ligase (400 units; New England Biolabs.). Ligation is performed for 2 h at 15°C. DNA ligation products are transformed into E. coli K12/803, ampicillin-resistant colonies selected and plasmid DNAs prepared using standard procedures (Sambrook et al., op. cit., Section 1.82-84 & 1.25-28). Plasmid containing the inserted ca. 630 bp XbaI DNA fragment in the required orientation for expression are detected by digestion using restriction endonuclease PstI. DNA from one such plasmid is prepared, and is referred to as 26-HuCκL1.

Example 9. Transfection of Sp2/O myeloma cells using 25-Huγ1H1 plus 25-HuCκL1 and 26-Huγ1H1/26-Huγ1H2 plus 26-HuCκL1

### 9.1 Growth and preparation of cells:

Sp2/0 cells are grown at 37°C in hybridoma medium (example 1.4). 24 h before transfection, Sp2/O cells are passaged into fresh growth medium at a concentration of 1 x 10$^5$ cells/ml. Immediately before transfection, cells are harvested as described above, and the cell pellets (total no. of cells ca. 9 x 10$^7$) washed twice in Dul-becco's PBS (without Ca$^{++}$, Mg$^{++}$; Seromed) at 4°C and resuspended in the same buffer at a concentration of ca. 1 x 10$^8$ cells/ml.

### 9.2 Preparation of DNA fragments and transfection:

Plasmid DNAs 25-Huγ1H1 (example 8.1), 25-HuCκL1 (example 8.2), 26-Huγ1H1 and 26-Huγ1H2 (example 8.3), and 26-HuCκL1 (example 8.4) are digested to termination using restriction endonuclease EcoRI to release the chimeric mouse:human Ig H- and L-chain insert DNA fragments. Digested DNA is extracted with phenol-/CHCl$_3$, then CHCl$_3$, precipitated at -20°C with ethanol in the presence of 0.3 M NaOAc, pH 7.0, washed with 70% ethanol at the same temperature, and resuspended in TE-buffer. Alternatively, intact plasmid DNAs can be used.

Samples (5 µg) of intact 25-Huγ1H1 and 25-HuCκL1 plasmid DNAs, or EcoRI-digested plasmid DNAs, are combined (total volume of 15 µl in TE-buffer) and added to 2 x 10$^7$ (200 µl) of Sp2/O cells at 0°C, previously grown, washed and resuspended in PBS-CM as described above (example 9.1). The cells plus DNA are drawn into the barrel of a TA750 electrotransfection apparatus (Kruess GmbH), pre-cooled to 0°C using ice-cold sterile PBS-CM. Cells are subjected to two electrical pulses of 3500 V/cm for 10 µs, with a 30 s interval between pulses, using the cylindrical electroporation chamber provided by the manufacturer. Cells are expelled gently into a clean, sterile cryotube (Nunc) and kept on ice for 10 min, after which they are diluted (1:3, v/v) into supplemented HB101™ growth medium (see above) containing 15% (v/v) FCS (Amimed) and incubated at room temperature for 20 min. The cells are then further diluted to 100 ml in the same growth medium and 1 ml samples distributed into wells of 24-well Nunclon™ tissue culture clusters (Delta). After incubation for 24 h at 37°C, as described above, 0.5 ml of the growth medium in each well are removed and replaced with 0.5 ml of prewarmed selection medium containing supplemented HB101™ (Hana Biologics) + 15% (v/v) FCS (Amimed) supplemented with a 1:45 dilution of HT (Sigma, 50 x HT media supplement), 0.5 µg/ml mycophenolic acid (Gibco) and 1 mg/ml geneticin (Gibco). One-half (0.5 ml) of the medium is replaced every 48 h with additional selection medium as described above, and selection continued for at least 14 days. In the case of chimeric gene constructs derived from the hybridoma 26-69-5, the L-chain construct 26-HuCκL1 is combined separately with each of the H-chain constructs 26-Huγ1H1 and 26-Huγ1H2, as described above.

The chimeric monoclonal antibodies MAb Ch25 containing 25-Huy1H1 and 25-HuCκL1 and MAb Ch26 containing 26-Huγ1H1 and 26-HuCκL1 are selected for further characterization. They show the same binding pattern as the antibody from which they are derived.

Example 10. Pharmaceutical preparation for parenteral application

10 mg chimeric monoclonal antibody Ch26 are dissolved in 50 ml PBS. The solution is passed through a bacteriological filter, the filtrate divided into 10 equal parts and filled in ampoules under aseptic conditions. The ampoules are preferably stored in the cold, e.g. at 4°C. This pharmaceutical preparation is suitable for injection.

Sequence listing

SEQ ID NO:1


SEQUENCE TYPE: nucleotide with corresponding protein
SEQUENCE LENGTH: 322 base pairs
MOLECULE TYPE: genomic DNA
ORIGINAL SOURCE ORGANISM: mouse
IMMEDIATE EXPERIMENTAL SOURCE: hybridoma cell line 25-57-1
NAME OF CELL CLONE: 25-LPCR1
FEATURES:          from 70 to 102 bp:               $CDR_{1L}$
                   from 148 to 168 bp:              $CDR_{2L}$
                   from 265 to 291 bp:              $CDR_{3L}$
PROPERTIES:        light chain variable region of anti-p24 antibody


```
GACATT CAG CTG ACC CAG TCT CCA GCC TCC CTA TCT GTA TCT   42
       Gln Leu Thr Gln Ser Pro Ala Ser Leu Ser Val Ser
                         5                   10

GTG GGA GAA ACT GTC ACC ATC ACA TGT CGT GCA AGT GAG AAT   84
Val Gly Glu Thr Val Thr Ile Thr Cys Arg Ala Ser Glu Asn
        15                  20                  25

ATT TAC AGT AAT TTA GCA TGG TAT CAG CAG AAA CAG GGA AAA  126
Ile Tyr Ser Asn Leu Ala Trp Tyr Gln Gln Lys Gln Gly Lys
            30                  35                      40

TCT CCT CAG CTC CTG GTC TAT GCT GCA ACA AAC TTA GCA GAT  168
Ser Pro Gln Leu Leu Val Tyr Ala Ala Thr Asn Leu Ala Asp
            45                  50

GGT GTG CCA TCA AGG TTC AGT GGC AGT GGA TCA GGC ACA CAG  210
Gly Val Pro Ser Arg Phe Ser Gly Ser Gly Ser Gly Thr Gln
 55                  60                  65

TAT TCC CTC AAG ATC AAC AGC CTG CAG TCT GAA GAT TTT GGG  252
Tyr Ser Leu Lys Ile Asn Ser Leu Gln Ser Glu Asp Phe Gly
    70                  75                  80

AGT TAT TAC TGT CAA CAT TTT TGG AGT ACT CCG TGG ACG TTC  294
Ser Tyr Tyr Cys Gln His Phe Trp Ser Thr Pro Trp Thr Phe
        85                  90                  95

GGT GGA GGC ACC AAG CTG GAG ATC TAAC                     322
Gly Gly Gly Thr Lys Leu Glu Ile
            100
```

SEQ ID NO: 2

SEQUENCE TYPE:  nucleotide with corresponding protein
SEQUENCE LENGTH:  338 base pairs
MOLECULE TYPE:  genomic DNA
ORIGINAL SOURCE ORGANISM:  mouse
IMMEDIATE EXPERIMENTAL SOURCE:  hybridoma cell line 25-57-1
NAME OF CELL CLONE:  25-HPCR1
FEATURES:        from 90 to 104 bp:      $CDR_{1H}$
                from 147 to 197 bp:     $CDR_{2H}$
                from 294 to 305 bp:     $CDR_{3H}$
PROPERTIES:      heavy chain variable region of anti-p24 antibody

```
AGGTCAAG CTG CAG CAG TCA GGG CCT GAG GTG GTG AGG CCT GGG   44
         Leu Gln Gln Ser Gly Pro Glu Val Val Arg Pro Gly
                      5                   10

GTC TCA GTG AAG ATT TCC TGC AAG GGT TCC GGC TAC ACA TTC    86
Val Ser Val Lys Ile Ser Cys Lys Gly Ser Gly Tyr Thr Phe
        15                  20                  25

ACT GAT TAT GCT ATG CAC TGG GTG AAA CAG AGT CAT ACA AAG   128
Thr Asp Tyr Ala Met His Trp Val Lys Gln Ser His Thr Lys
                30                  35                  40

AGT CTA GAG TGG ATT GGA ATT ATT AGG ACT TAC AAT GGT AAT   170
Ser Leu Glu Trp Ile Gly Ile Ile Arg Thr Tyr Asn Gly Asn
                    45                  50

ACA AAC TAC AAC CAG AAG TTT AAG GGC AAG GCC ACA ATG ACT   212
Thr Asn Tyr Asn Gln Lys Phe Lys Gly Lys Ala Thr Met Thr
55                  60                  65

GTA GAC AAA TCC TCC AGC ACA GCC TAT ATG GAA CTT GCC AGA   254
Val Asp Lys Ser Ser Ser Thr Ala Tyr Met Glu Leu Ala Arg
    70              75                  80

TTG ACA TCT GAG GAT TCT GCC ATC TAT TAC TGT GCA AGC AAC   296
Leu Thr Ser Glu Asp Ser Ala Ile Tyr Tyr Cys Ala Ser Asn
        85                  90                  95

GTT GCT TAC TGG GGC CAA GGG ACC ACG GTC ACC GTCTCCTCA    338
Val Ala Tyr Trp Gly Gln Gly Thr Thr Val Thr
        100                 105
```

SEQ ID NO:3

SEQUENCE TYPE: nucleotide with corresponding protein
SEQUENCE LENGTH: 340 base pairs
MOLECULE TYPE: genomic DNA
ORIGINAL SOURCE ORGANISM: mouse
IMMEDIATE EXPERIMENTAL SOURCE: hybridoma cell line 26-69-5
NAME OF CELL CLONE: 26-LPCR1
FEATURES:       from 70 to 120 bp:            CDR$_{4L}$
                from 166 to 186 bp:           CDR$_{5L}$
                from 283 to 309 bp:           CDR$_{6L}$
PROPERTIES: light chain variable region of anti-p24 antibody


```
GACATT CAG CTG ACC CAG TCT CCA TCC TCC CTA GCT GTG TCA    42
       Gln Leu Thr Gln Ser Pro Ser Ser Leu Ala Val Ser
                    5                   10


GTT GGA GAG AAG GTT ACT ATG AGC TGC AAG TCC AGT CAG AGC    84
Val Gly Glu Lys Val Thr Met Ser Cys Lys Ser Ser Gln Ser
        15                  20                  25


CTT TTA TAT AGT AGC AAT CAA AAG AAC TAC TTG GCC TGG TAC   126
Leu Leu Tyr Ser Ser Asn Gln Lys Asn Tyr Leu Ala Trp Tyr
                30                  35                  40


CAG CAG AAA CCA GGG CAG TCT CCT AAA CTG CTG ATT TAC TGG   168
Gln Gln Lys Pro Gly Gln Ser Pro Lys Leu Leu Ile Tyr Trp
                45                  50


GCA TCC ACT AGG GAA TCT GGG GTC CCT GAT CGC TTC ACA GGC   210
Ala Ser Thr Arg Glu Ser Gly Val Pro Asp Arg Phe Thr Gly
55                  60                  65


AGT GGA TCT GGG ACA GAT TTC ACT CTC ACC ATC AGC AGT GTG   252
Ser Gly Ser Gly Thr Asp Phe Thr Leu Thr Ile Ser Ser Val
        70                  75                  80


AAG GCT GAA GAC CTG GCA GTT TAT TAC TGT CAG CAA TAT TAT   294
Lys Ala Glu Asp Leu Ala Val Tyr Tyr Cys Gln Gln Tyr Tyr
        85                  90                  95


AGC TAT CCG TGG ACG TTC GGT GGA GGC ACC AAG CTG GAG ATC   336
Ser Tyr Pro Trp Thr Phe Gly Gly Gly Thr Lys Leu Glu Ile
            100                 105                 110


TAAC                                                       340
```

SEQ ID NO:4

SEQUENCE TYPE:  nucleotide with corresponding protein
SEQUENCE LENGTH:  368 base pairs
MOLECULE TYPE:  genomic DNA
ORIGINAL SOURCE ORGANISM:  mouse
IMMEDIATE EXPERIMENTAL SOURCE:  hybridoma cell line 26-69-5
NAME OF CELL CLONE:  26-HPCR1
FEATURES:          from 90 to 104 bp:              $CDR_{4H}$
                   from 147 to 197 bp:             $CDR_{5H}$
                   from 294 to 335 bp:             $CDR_{6H}$
PROPERTIES:  heavy chain variable region of anti-p24 antibody


```
AGGTCAAG CTG CAG CAG TCT GGA GCG GAC GTG ATG AAG CCT GGG   44
        Leu Gln Gln Ser Gly Ala Asp Val Met Lys Pro Gly
                          5                 10


GCC TCA GTG AAG ATC TCC TGC AAG ACT ACT GGC TAC ACA TTC   86
Ala Ser Val Lys Ile Ser Cys Lys Thr Thr Gly Tyr Thr Phe
        15                  20                  25


AGT ATG TAC TGG TTA GAG TGG GTA AAG CAG AGG CCT GGA CAT  128
Ser Met Tyr Trp Leu Glu Trp Val Lys Gln Arg Pro Gly His
            30                  35                      40


GGC CTT GAG TGG ATT GGA GAG ATT TCA CCT GGA ACT TTT ACT  170
Gly Leu Glu Trp Ile Gly Glu Ile Ser Pro Gly Thr Phe Thr
                45                  50


ACT AAC TAC AAT GAG AAA TTC AAG GCC AAG GCC ACA TTC ACT  212
Thr Asn Tyr Asn Glu Lys Phe Lys Ala Lys Ala Thr Phe Thr
 55                  60                  65


GCG GAT ACA TCC TCC AAC ACA GCC TAC CTG CAA CTC AGC GGC  254
Ala Asp Thr Ser Ser Asn Thr Ala Tyr Leu Gln Leu Ser Gly
    70                  75                  80


CTG ACA TCT GAG GAC TCT GCC GTC TAC TTC TGT GCA AGA TTC  296
Leu Thr Ser Glu Asp Ser Ala Val Tyr Phe Cys Ala Arg Phe
        85                  90                  95


TCC CAT TAT TCC GGT AAT AAC TAC GAC TAC TTT GAC TAC TGG  338
Ser His Tyr Ser Gly Asn Asn Tyr Asp Tyr Phe Asp Tyr Trp
            100                 105                 110


GGC CAA GGG ACC ACG GTC ACC GTCTCCTCA                     368
Gly Gln Gly Thr Thr Val Thr
                115
```

SEQ ID NO:5

SEQUENCE TYPE: nucleotide with corresponding protein
SEQUENCE LENGTH: 368 base pairs
MOLECULE TYPE: genomic DNA
ORIGINAL SOURCE ORGANISM: mouse
IMMEDIATE EXPERIMENTAL SOURCE: hybridoma cell line 26-69-5
NAME OF CELL CLONE: 26-HPCR2
FEATURES:          from 90 to 104 bp:          $CDR_{7H}$
                   from 147 to 197 bp:          $CDR_{8H}$
                   from 294 to 335 bp:          $CDR_{9H}$
PROPERTIES: heavy chain variable region of anti-p24 antibody

```
AGGTGCAG CTG CAG CAG TCT GGG GCT GAA CTG GCA AAA CCT GGG  44
         Leu Gln Gln Ser Gly Ala Glu Leu Ala Lys Pro Gly
                          5                    10

GCC TCA GTG AAA ATG TCC TGC AAG GCT TCT GGC TAC ACC TTT  86
Ala Ser Val Lys Met Ser Cys Lys Ala Ser Gly Tyr Thr Phe
         15                  20                  25

ACT TCC TAC TGG ATG CAC TGG GTA AAA CAG AGG CCT GGA CAG  128
Thr Ser Tyr Trp Met His Trp Val Lys Gln Arg Pro Gly Gln
             30                  35                  40

GGT CTG GAA TGG ATT GGA TAC ATT AAT CCT AGC ACT GGT TAT  170
Gly Leu Glu Trp Ile Gly Tyr Ile Asn Pro Ser Thr Gly Tyr
                 45                  50

ACT GAG TAC AAT CAG AAG TTC AAG GAC AAG GCC ACA TTG ACT  212
Thr Glu Tyr Asn Gln Lys Phe Lys Asp Lys Ala Thr Leu Thr
 55                  60                  65

GCA GAC AAA TCC TCC AGC ACA GCC TAC ATG CAA CTG AGC AGC  254
Ala Asp Lys Ser Ser Ser Thr Ala Tyr Met Gln Leu Ser Ser
     70                  75                  80

CTG ACA TCT GAG GAC TCT GCA GTC TAT TAC TGT GCA AGA TGG  296
Leu Thr Ser Glu Asp Ser Ala Val Tyr Tyr Cys Ala Arg Trp
         85                  90                  95

GGA TAT TCC ACT CAC TGG GAC CCT TAT ACT TTG GAC TAC TGG  338
Gly Tyr Ser Thr His Trp Asp Pro Tyr Thr Leu Asp Tyr Trp
             100                 105                 110

GGC CAA GGG ACC ACG GTC ACC GTCTCCTCA                     368
Gly Gln Gly Thr Thr Val Thr
             115
```

## Claims

1. A monoclonal antibody directed against HIV core protein p24 which recognizes p24 expressed on the surface of HIV-infected macrophages, and derivatives thereof which retain the specificity of the antibody from which they are derived.

2. A monoclonal antibody according to claim 1 which kills HIV-infected cells, and derivatives thereof.

3. A monoclonal antibody according to claim 1 or 2 which prevents the spread of infection from HIV-infected cells to non-infected cells, and derivatives thereof.

4. A monoclonal antibody according to any of the claims 1 to 3 which reduces the amount of infectious HIV produced by macrophages and chronically infected lymphoid cells, and derivatives thereof.

5. A monoclonal antibody according to any of the claims 1 to 4 which is a murine antibody, and derivatives thereof.

6. The monoclonal antibody according to claim 5 with the designation MAb 25-57-1, and derivatives thereof.

7. The monoclonal antibody according to claim 5 with the designation MAb 26-69-5, and derivatives thereof.

8. A chimeric monoclonal antibody consisting of human constant regions and murine variable regions of an antibody according to claim 5, and derivatives thereof.

9. The chimeric monoclonal antibody according to claim 8 designated Ch25 which contains the variable regions of the murine monoclonal antibody 25-57-1, and derivatives thereof.

10. The chimeric monoclonal antibody according to claim 8 designated Ch26 which contains the variable regions of the murine monoclonal antibody 26-69-5, and derivatives thereof.

11. A derivative of a monoclonal antibody according to any of the claims 1 to 10 which is an antibody fragment, which is a conjugate of the antibody or antibody fragment with an enzyme, with a fluorescent marker, with a chemiluminescent marker, with a metal chelate, with avidin, or with biotin or the like, or which is a radioactively labelled antibody or antibody fragment.

12. A process for the preparation of a monoclonal antibody or a derivative thereof according to any of claims 1 to 11, characterized in that a mammalian cell producing such antibody is multiplied in vitro or in vivo and, when required, the obtained monoclonal antibody is isolated and/or converted into a derivative thereof.

13. A recombinant DNA comprising an insert coding for a light chain murine variable region and/or for a heavy chain murine variable region of a chimeric antibody according to claim 8.

14. A recombinant DNA according to claim 13 comprising an insert coding for a light chain murine variable region of a chimeric antibody, fused to a human constant region $\kappa$ or $\lambda$, and/or an insert coding for a heavy chain murine variable region of a chimeric antibody, fused to a human constant region $\gamma$.

15. A recombinant DNA according to claim 13 which is a hybrid vector comprising an insert coding for a light chain murine variable region of a chimeric antibody, fused to a human constant region $\kappa$ or $\lambda$ and/or an insert coding for a heavy chain murine variable region of a chimeric antibody, fused to a human constant region $\gamma$, an origin of replication or an autonomously replicating sequence, one or more dominant marker sequences and, optionally, expression control sequences, signal sequences and additional restriction sites.

16. A process for the preparation of a recombinant DNA according to any of claims 13 to 15 comprising the steps of
a) isolating murine DNAs from a suitable hybridoma cell line and selecting the desired DNAs coding for the variable regions of monoclonal antibodies with the desired specificity using DNA probes,
b) isolating human DNAs from a genomic library and selecting the desired DNAs coding for the constant regions of monoclonal antibodies using DNA probes,
c) constructing chimeric mouse/human genes by incorporating the DNA of step a) and b) into appropriate hybrid vectors,
d) transferring the obtained hybrid vectors into a recipient host cell or refering the DNA coding for the chimeric mouse/human genes and transferring the unlinked DNA into a recipient host cell,
e) selecting and culturing the transformed host cell, and
f) optionally isolating the desired DNA.

17. A continuous cell line which secretes monoclonal antibodies according to any of the claims 1 to 10.

18. A process for the preparation a continuous cell line according to claim 17 wherein

a) a suitable mammal is immunized with HIV core protein p24, optionally mixed with an adjuvant, antibody-producing cells of this mammal are fused with cells of a continuous cell line, the hybrid cells obtained in the fusion are cloned, and cell clones secreting the desired monoclonal antibodies are selected, or

b) suitable host cells are transformed with a recombinant DNA, transformed cells are cloned, and cell clones secreting the desired chimeric monoclonal antibodies are selected.

19. A method of preventing the progression of AIDS or of treating HIV infection by administering a therapeutically effective amount of a monoclonal antibody or of a derivative thereof according to any of the claims 1 to 11.

20. A pharmaceutical composition for prevention of the progression of AIDS or the treatment of HIV-infection comprising a therapeutically effective amount of a monoclonal antibody and/or a derivative thereof according to any of the claims 1 to 11, and a pharmaceutically acceptable carrier.

21. Use of a monoclonal antibody and/or of a derivative thereof according to any of the claims 1 to 11 for the qualitative and/or quantitative determination of HIV core protein p24.

22. A test kit for the qualitative and/or quantitative determination of HIV core protein p24 comprising monoclonal antibodies and/or a derivative thereof according to any of the claims 1 to 11 and, optionally, other polyclonal or monoclonal antibodies and/or adjuncts.

**Claims for the following Contracting State: ES**

1. A process for the preparation of a monoclonal antibody directed against HIV core protein p24 which recognizes p24 expressed on the surface of HIV-infected macrophages, and derivatives thereof which retain the specificity of the antibody from which they are derived, characterized in that a mammalian cell producing such antibody is multiplied in vitro or in vivo and, when required, the obtained monoclonal antibody is isolated and/or converted into a derivative thereof.

2. A process according to claim 1 wherein the monoclonal antibody or derivative thereof kills HIV-infected cells.

3. A process according to claim 1 or 2 wherein the monoclonal antibody or derivative thereof prevents the spread of infection from HIV-infected cells to non-infected cells.

4. A process according to any of the claims 1 to 3 wherein the monoclonal antibody or derivative thereof reduces the amount of infectious HIV produced by macrophages and chronically infected lymphoid cells.

5. A process according to any of the claims 1 to 4 wherein the monoclonal antibody or derivative thereof is a murine antibody or a derivative of a murine antibody.

6. A process according to any of the claims 1 to 5 for the preparation of the monoclonal antibody with the designation MAb 25-57-1, and derivatives thereof.

7. A process according to any of the claims 1 to 5 for the preparation of the monoclonal antibody with the designation MAb 26-69-5, and derivatives thereof.

8. A process for the preparation of a chimeric monoclonal antibody consisting of human constant regions and murine variable regions of an antibody prepared by a process according to claim 5, and of derivatives of such a chimeric antibody.

9. A process according to claim 8 for the preparation of the chimeric monoclonal antibody designated Ch25 which contains the variable regions of the murine monoclonal antibody 25-57-1, and derivatives thereof.

10. A process according to claim 8 for the preparation of the chimeric monoclonal antibody designated Ch26 which contains the variable regions of the murine monoclonal antibody 26-69-5, and derivatives thereof.

11. A process according to any of the claims 1 to 10 wherein the derivative is an antibody fragment, a conjugate of the antibody or antibody fragment with an enzyme, with a fluorescent marker, with a chemiluminescent marker, with a metal chelate, with avidin, or with biotin or the like, or a radioactively labelled antibody or antibody fragment.

12. A process for the preparation of a recombinant DNA comprising an insert coding for a light chain murine variable region and/or for a heavy chain murine variable region of a chimeric antibody prepared by a process according to claim 8, comprising the steps of
   a) isolating murine DNAs from a suitable hybridoma cell line and selecting the desired DNAs coding for the variable regions of monoclonal antibodies with the desired specificity using DNA probes,
   b) isolating human DNAs from a genomic library and selecting the desired DNAs coding for the constant regions of monoclonal antibodies using DNA probes,
   c) constructing chimeric mouse/human genes by incorporating the DNA of step a) and b) into appropriate hybrid vectors,
   d) transferring the obtained hybrid vectors into a recipient host cell or retrieving the DNA coding for the chimeric mouse/human genes and transferring the unlinked DNA into a recipient host cell,
   e) selecting and culturing the transformed host cell, and
   f) optionally isolating the desired DNA.

13. A process according to claim 12 wherein the recombinant DNA comprises an insert coding for a light chain murine variable region of a chimeric antibody, fused to a human constant region $\kappa$ or $\lambda$, and/or an insert coding for a heavy chain murine variable region of a chimeric antibody, fused to a human constant region $\gamma$.

14. A process according to claim 12 wherein the recombinant DNA is a hybrid vector comprising an insert coding for a light chain murine variable region of a chimeric antibody, fused to a human constant region $\kappa$ or $\lambda$ and/or an insert coding for a heavy chain murine variable region of a chimeric antibody, fused to a human constant region $\gamma$, an origin of replication or an autonomously replicating sequence, one or more dominant marker sequences and, optionally, expression control sequences, signal sequences and additional restriction sites.

15. A continuous cell line which secretes monoclonal antibodies prepared by a process according to any of the claims 1 to 10.

16. A process for the preparation a continuous cell line according to claim 15 wherein
   a) a suitable mammal is immunized with HIV core protein p24, optionally mixed with an adjuvant, antibody-producing cells of this mammal are fused with cells of a continuous cell line, the hybrid cells obtained in the fusion are cloned, and cell clones secreting the desired monoclonal antibodies are selected, or
   b) suitable host cells are transformed with a recombinant DNA, transformed cells are cloned, and cell clones secreting the desired chimeric monoclonal antibodies are selected.

17. A process for the preparation of a pharmaceutical composition, characterized in that a monoclonal antibody and/or a derivative thereof prepared by a process according to any of the claims 1 to 11 is mixed with a pharmaceutically acceptable carrier.

**Claims for the following Contracting State: GR**

1. A process for the preparation of a monoclonal antibody directed against HIV core protein p24 which recognizes p24 expressed on the surface of HIV-infected macrophages, and derivatives thereof which retain the specificity of the antibody from which they are derived, characterized in that a mammalian cell producing such antibody is multiplied in vitro or in vivo and, when required, the obtained monoclonal antibody is isolated and/or converted into a derivative thereof.

2. A process according to claim 1 wherein the monoclonal antibody or derivative thereof kills HIV-infected cells.

3. A process according to claim 1 or 2 wherein the monoclonal antibody or derivative thereof prevents the spread of infection from HIV-infected cells to non-infected cells.

4. A process according to any of the claims 1 to 3 wherein the monoclonal antibody or derivative thereof reduces the amount of infectious HIV produced by macrophages and chronically infected lymphoid cells.

5. A process according to any of the claims 1 to 4 wherein the monoclonal antibody or derivative thereof is a murine antibody or a derivative of a murine antibody.

6. A process according to any of the claims 1 to 5 for the preparation of the monoclonal antibody with the designation MAb 25-57-1, and derivatives thereof.

7. A process according to any of the claims 1 to 5 for the preparation of the monoclonal antibody with the designation MAb 26-69-5, and derivatives thereof.

8. A process for the preparation of a chimeric monoclonal antibody consisting of human constant regions and murine variable regions of an antibody prepared by a process according to claim 5, and of derivatives of such a chimeric antibody.

9. A process according to claim 8 for the preparation of the chimeric monoclonal antibody designated Ch25 which contains the variable regions of the murine monoclonal antibody 25-57- 1, and derivatives thereof.

10. A process according to claim 8 for the preparation of the chimeric monoclonal antibody designated Ch26 which contains the variable regions of the murine monoclonal antibody 26-69-5, and derivatives thereof.

11. A process according to any of the claims 1 to 10 wherein the derivative is an antibody fragment, a conjugate of the antibody or antibody fragment with an enzyme, with a fluorescent marker, with a chemiluminescent marker, with a metal chelate, with avidin, or with biotin or the like, or a radioactively labelled antibody or antibody fragment.

12. A recombinant DNA comprising an insert coding for a light chain murine variable region and/or for a heavy chain murine variable region of a chimeric antibody prepared by a process according to claim 8.

13. A recombinant DNA according to claim 12 comprising an insert coding for a light chain murine variable region of a chimeric antibody, fused to a human constant region $\kappa$ or $\lambda$, and/or an insert coding for a heavy chain murine variable region of a chimeric antibody, fused to a human constant region $\gamma$.

14. A recombinant DNA according to claim 12 which is a hybrid vector comprising an insert coding for a light chain murine variable region of a chimeric antibody, fused to a human constant region $\kappa$ or $\lambda$ and/or an insert coding for a heavy chain murine variable region of a chimeric antibody, fused to a human constant region $\gamma$, an origin of replication or an autonomously replicating sequence, one or more dominant marker sequences and, optionally, expression control sequences, signal sequences and additional restriction sites.

15. A process for the preparation of a recombinant DNA according to any of claims 12 to 14 comprising the steps of
a) isolating murine DNAs from a suitable hybridoma cell line and selecting the desired DNAs coding for the variable regions of monoclonal antibodies with the desired specificity using DNA probes,
b) isolating human DNAs from a genomic library and selecting the desired DNAs coding for the constant regions of monoclonal antibodies using DNA probes,
c) constructing chimeric mouse/human genes by incorporating the DNA of step a) and b) into appropriate hybrid vectors,
d) transferring the obtained hybrid vectors into a recipient host cell or retrieving the DNA coding for the chimeric mouse/human genes and transferring the unlinked DNA into a recipient host cell,
e) selecting and culturing the transformed host cell, and
f) optionally, isolating the desired DNA.

16. A continuous cell line which secretes monoclonal antibodies prepared by a process according to any of the claims 1 to 10.

17. A process for the preparation a continuous cell line according to claim 16 wherein
a) a suitable mammal is immunized with HIV core protein p24, optionally mixed with an adjuvant, antibody-producing cells of this mammal are fused with cells of a continuous cell line, the hybrid cells obtained in the fusion are cloned, and cell clones secreting the desired monoclonal antibodies are selected, or

b) suitable host cells are transformed with a recombinant DNA, transformed cells are cloned, and cell clones secreting the desired chimeric monoclonal antibodies are selected.

18. A process for the preparation of a pharmaceutical composition, characterized in that a monoclonal antibody and/or a derivative thereof prepared by a process according to any of the claims 1 to 11 is mixed with a pharmaceutically acceptable carrier.

EP 0 519 866 A1

**European Patent Office**

**PARTIAL EUROPEAN SEARCH REPORT**

which under Rule 45 of the European Patent Convention shall be considered, for the purposes of subsequent proceedings, as the European search report

Application Number

EP 92 81 0445

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-9 107 494 (XOMA CORPORATION) * Page 9, line 16 - page 18; page 35, line 9 - page 43 * | 1-22 | C 12 N 15/13 C 12 P 21/08 C 12 N 15/63 C 12 N 5/10 C 12 N 5/20 A 61 K 39/42 G 01 N 33/569 G 01 N 33/577 |
| X | WO-A-9 107 493 (XOMA CORPORATION) * Page 7, line 29 - page 16, line 6; page 30, line 17 - page 38, line 4 * | 1-22 | |
| X | WO-A-8 904 376 (COULTER CORPORATION) * Claims * | 1-7,11, 12,17, 18,21, 22 | |
| D,X | EP-A-0 345 461 (ABBOTT LABORATORIES) * Claims * | 1-7,11, 12,17, 18,21, 22 | |
| D,X | US-A-4 843 011 (SARNGADHARAN et al.) | 1-7,11, 12,17- 22 | |

-/-

**TECHNICAL FIELDS SEARCHED (Int. Cl.5)**

C 12 P
C 12 N
A 61 K
G 01 N
C 07 K

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

Remark: Although claim 19 is directed to a method of treatment of the human/animal body (Art. 52(4) EPC) the search has been carried out and based on the alleged effects of the compound/composition

| Place of search | Date of completion of the search | Examiner |
|---|---|---|
| THE HAGUE | 17-09-1992 | NOOIJ F.J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**PARTIAL EUROPEAN SEARCH REPORT**

Application Number

EP  92 81 0445

| Category | Citation of document with indication, where appropriate, of relevant passages | Relevant to claim | CLASSIFICATION OF THE APPLICATION (Int. Cl.5) |
|---|---|---|---|
| D,Y | * Column 5, line 36 - column 6, line 60 * <br> --- | 8-10,13 -16 | |
| Y | NATURE, vol. 332, 24 March 1988, LONDON, GB, pages 323-327; L. RIECHMANN et al.: "Reshaping human antibodies for therapy." <br> * The whole document * <br> ----- | 8-10,13 -16 | |
|  |  |  | TECHNICAL FIELDS SEARCHED (Int. Cl.5) |

EPO FORM 1503 03.82 (P0410)